(19) Europäisches Patentamt
European Patent Office
Office européen des brevets

(11) **EP 3 366 216 A1**

(12) **EUROPEAN PATENT APPLICATION**

(43) Date of publication:
**29.08.2018 Bulletin 2018/35**

(51) Int Cl.:
***A61B 6/00*** (2006.01)          ***A61B 5/22*** (2006.01)
***G06F 19/00*** (2018.01)

(21) Application number: **18168090.1**

(22) Date of filing: **19.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2014 US 201414184042**

(62) Document number(s) of the earlier application(s) in accordance with Art. 76 EPC:
**14882988.0 / 3 107 451**

(71) Applicant: **LUMIRADX UK LTD**
**London**
**SE1 2AQ (GB)**

(72) Inventors:
• **BLACKADAR, Thomas P**
**Hanson, MA 02341 (US)**
• **MONAHAN, David P**
**West Chester, Pennsylvania 19382 (US)**
• **GAUDET, Paul J**
**Dracut, Massachusetts 01826 (US)**
• **QUINLAN, Thomas J**
**Stow, Massachusetts 01775 (US)**

(74) Representative: **HGF Limited**
**1 City Walk**
**Leeds LS11 9DX (GB)**

(54) **HEALTH MONITOR**

(57)     A health monitor configured to be supported by a subject, which is configured to receive motion data generated by an accelerometer of the health monitor in response to the activity performed by a subject over a sequence of time intervals and to receive cardiac data from a cardiac sensor of the health monitor detected during performance of the activity over the sequence of time intervals. The health monitor calculates a caloric burn rate for the activity performed by the subject over each time interval according to an algorithm based upon information in both the motion data and cardiac data. The health monitor records a first credit value for each time interval that the caloric burn rate falls within a first recommended range for the duration of that time interval and records a second credit value for each time interval that the caloric burn rate falls within a second recommended range for the duration of that time interval.

FIG. 7A

EP 3 366 216 A1

**Description**

RELATED APPLICATIONS

This application is a continuation-in-part of U.S. Application Ser. No. 13/840,098, titled "Versatile Sensors with Data Fusion Functionality," filed on March 15, 2013, which is a continuation-in-part of U.S. Application Ser. No. 13/690,313, titled "Intelligent Activity Monitoring," filed on November 30, 2012, which claims the benefit of U.S. provisional Application Ser. No. 61/566,528, titled "Intelligent Activity Monitoring," filed on December 2, 2011. The entire disclosures of the foregoing applications are incorporated herein by reference.

FIELD

This disclosure relates generally to apparatuses and methods for monitoring physical activity of a subject and determining a level of health benefit from the detected activity.

BACKGROUND

There currently exist small sensors that can be worn by a user to monitor a physical activity performed by the user or two similar types of user activity. As an example, the FitLinxx® ActiPed+ (available from FitLinxx, Shelton, CT, USA) is a small device that can be clipped to a shoe and used to monitor walking and running activities by the user. When a user walks or runs, an on-board accelerometer outputs data that is stored on the device for subsequent transmission to a computer system. The computer system can analyze the data to determine activity type, and calculate various activity parameters (*e.g.,* duration of activity, total steps, distance traveled, and calories burned). Results of the data analysis may be presented on the computer's display, so that a user may review details of his or her activity. The results may be stored, so that the user can maintain a record of exercise regimens and track progress toward exercise goals or so that the data may be used by medical personnel to track recovery from an illness or injury. Other modern activity monitors perform similar functions with varying degrees of accuracy.

Currently, vendors and users of pedometers and activity monitors consider 10,000 steps per day to be a healthful amount of activity. This data has been amassed over time, but has been based in part on incorrect step counting throughout a subject's day. The literature frequently reports inaccuracies in step counting by various pedometers and algorithms used. Although manufacturers continually try to improve the accuracy of their devices, many devices can be highly inaccurate depending on a user's activity patterns. For example, activity monitors detect and count steps differently. Some activity monitors pick up "steps" from activities that include driving, swiveling in a chair, restless leg syndrome, eating (for some wrist worn devices), and riding on a train or bus. These add to a baseline of inaccurate step counts during a day. To make an all-day activity monitor correctly count steps and ignore all other motions may require multiple inputs and may require more expensive, more sophisticated, and more power hungry devices. Such devices, although they may be highly accurate, may be too expensive for many consumers.

SUMMARY OF EXAMPLE EMBODIMENTS

The inventors have appreciated that although a record of steps, distance traveled, duration of activity, or calories burned are useful to many users for maintaining a log of physical activities, the usefulness of the data can be further improved by computing, from data representative of the activity, levels or units of health benefits received by the user as a result of the activity. For example, two people taking the same number of steps in a day, traveling the same distance, and taking the same amount of time to do so may receive markedly different health benefits from their physical activities. For example, an overweight, aged individual may receive more health benefit from the activity than a young, fit individual. The inventors have recognized that in some settings (*e.g.,* medical diagnosis and treatment), it may be more relevant to assess the quality, or health benefit level, of activity performed rather than a raw number of steps, distance traveled, calories burned, or duration of activity. In some implementations, units of health benefits may be standardized to provide more accurate analysis of fitness benefits across populations and across fitness devices.

The inventors have developed methods and apparatus that may be used to determine levels of health benefits from activities detected by wearable activity or health monitors. The determination of health benefits may be tied to recognizable health standards established by a health entity, the Center for Disease Control or World Health Organization for example. According to some embodiments, health benefit levels of performed activities may be determined from a combination of data that may include, but not be limited to, activity type, continuous time in the activity, vigorousness of the activity, and exercise guidelines established by the health entity. By linking the health benefits to recognizable health standards and using certain criteria for establishing health-creditable activity, the inventors have developed a system that more accurately reflects the efficacy of exercise detected by wearable health monitors.

In some implementations, accuracy of a health monitor may be improved using cardia data from a subject in addition to motion data representative of an activity performed by the subject. For example, heart rate data in combination with motion data may be used to improve the accuracy of caloric burn for an activity over a time interval, and thereby improve the accuracy of vigorousness level for the activity. The type of activity may, or may not, be identified by the health monitor.

According to some embodiments, a health monitor is configured to be supported by a subject and may comprise an accelerometer configured to generate motion data in response to a first activity performed by the subject, a cardiac sensor configured to detect at least a heart rate of the subject during performance of the first activity, an on-board power source configured to provide power to the accelerometer and the cardiac sensor, and a processor configured to calculate a first caloric burn rate for the first activity performed by the subject according to a first method based upon data from both the accelerometer and the cardiac sensor.

In some aspects, the processor is further configured to determine at least one power conservation mode of the health monitor based upon data received from the cardiac sensor. The at least one power conservation mode may, for example, comprise a mode in which power is reduced to at least the cardiac sensor for an interval between T-wave and P-wave portions of successive cardiac cycles.

In some implementations, the processor is configured to calculate the first caloric burn rate using values of heart rate and speed of the subject, or includes values of heart rate and foot contact time of the subject. According to some aspects, the processor is configured to calculate the first caloric burn rate using values of heart rate and a $VO_2$ max value computed for the subject.

In some aspects, a health monitor's processor may further be configured to calibrate a second method for determining caloric burn for a subject performing an activity, wherein the calibration is based upon results obtained from the first method. The second method may, for example, comprise determining caloric burn based upon heart rate and not based upon data from the accelerometer.

According to some implementations, a health monitor may further comprise a timer, and be configured to calculate a second caloric burn rate for a second activity performed by the subject for a first time interval of a sequence of first time intervals, and determine whether the second caloric burn rate falls within at least one recommended range of values proscribed by a health entity for the duration of the first time interval. The health entity may be the Center for Disease Control or the World Health Organization. In some aspects, the health entity may be a physician, a qualified medical personnel, or a professional trainer.

According to some aspects, a processor of a health monitor may be configured to verify performance of the second activity from a power spectrum of the motion data. Additionally, or alternatively the processor may be configured to determine the intensity level of the second activity from a heart rate and/or respiratory rate of the subject.

In some aspects, the processor of a health monitor may be further configured to record a first credit value for each first time interval that the caloric burn rate falls within a first recommended range of the at least one recommended range for the duration of the first time interval, and record a second credit value for each first time interval that the caloric burn rate falls within a second recommended range of the at least one recommended range for the duration of the first time interval. In some implementations, the first recommended range of the at least one recommended range may be between approximately 3.5 kilocalories per minute and approximately 7 kilocalories per minute and the second recommended range of the at least one recommended range includes values greater than approximately 7 kilocalories per minute. In some aspects, the processor may be further configured to identify a type of the second activity performed by the subject from among a plurality of activity types for which the health monitor is configured to recognize, and may identify non-human activities.

According to some implementations, a processor of a health monitor may be further configured to determine, from data received from the accelerometer, a period of increased activity performed by the subject, and determine a heart-rate recovery time following cessation of the activity. In some aspects, the processor may be further configured to calculate heart rate variability or a LF/HF spectral power ratio from data received from the cardiac sensor.

In some implementations, a health monitor includes two electrodes configured to be electrically connected to the subject using a removable adhesive. In some aspects, an accelerometer of a health monitor comprises a three-axis accelerometer.

The foregoing aspects, features, and implementations may be used in any suitable combination in one or more embodiments of a health monitor.

Also contemplated are methods for operating a health monitor. According to some embodiments, a method for determining fitness metrics for a subject by a health monitor configured to be supported by the subject may comprise acts of receiving, by a processor, motion data that was generated by the health monitor in response to a first activity performed by the subject. A method may further include receiving, from a cardiac sensor in communication with the processor, cardiac data for the subject detected during performance of the first activity, and calculating a first caloric burn rate for the first activity performed by the subject according to a first algorithm based upon information in both the motion data and cardiac data.

According to some implementations, a method may further comprise executing a power conservation mode of the health monitor based upon data received from the cardiac sensor. In some aspects, a method may further comprise reducing power to at least the cardiac sensor for an interval between T-wave and P-wave portions of successive cardiac cycles.

In some aspects, the calculation of the first caloric burn rate includes values of heart rate and speed of the subject or values of heart rate and foot contact time of the subject. In some implementations, the calculation of the first caloric burn rate includes values of heart rate and a $VO_2$ max value computed for the subject.

According to some aspects, a method may further comprise calibrating a second algorithm for determining caloric burn for a subject performing an activity, wherein the calibration is based upon results obtained from the first algorithm. In some aspects, the second algorithm comprises determining caloric burn based upon cardiac data and not based upon motion data.

In some implementations, a method for operating a health monitor comprises calculating a second caloric burn rate for a second activity performed by the subject for a first time interval of a sequence of first time intervals, and determining whether the second caloric burn rate falls within at least one recommended range of values proscribed by a health entity for the duration of the first time interval. In some aspects, the health entity is the Center for Disease Control or the World Health Organization.

According to some implementations, a method may further comprise determining a power spectrum for the motion data, and verifying performance of the second activity from the power spectrum. In some aspects, a method further comprises recording a first credit value for each first time interval that the second caloric burn rate falls within a first recommended range of the at least one recommended range for the duration of the first time interval, and recording a second credit value for each first time interval that the second caloric burn rate falls within a second recommended range of the at least one recommended range for the duration of the first time interval. In some aspects, the first recommended range of the at least one recommended range is between approximately 3.5 kilocalories per minute and approximately 7 kilocalories per minute and the second recommended range of the at least one recommended range includes values greater than approximately 7 kilocalories per minute. A method may further comprise identifying a type of the second activity performed by the subject from among a plurality of activity types for which the health monitor is configured to recognize.

According to some implementations, a method for operating a health monitor may further comprise determining, from the motion data, a period of increased activity performed by the subject, and determining a heart-rate recovery time following cessation of the activity. In some implementations, a method further comprises calculating heart rate variability or a LF/HF spectral power ratio from the cardiac data.

The foregoing aspects and implementations of various acts and features may be combined in any suitable manner in one or more embodiments of operating a health monitor.

Also contemplated are tangible storage devices or computer-readable medium that include machine-readable instructions that, when executed by at least one processor of a health monitor that includes a cardiac sensor and accelerometer, adapt the health monitor to execute any one or combination of the aforementioned acts of operating a health monitor. For example, a storage device may include machine-readable instructions that adapt the health monitor to receive, by the processor, motion data that was generated by the accelerometer in response to a first activity performed by a subject, receive cardiac data for the subject detected during performance of the first activity, and calculate a first caloric burn rate for the first activity performed by the subject according to a first algorithm based upon information in both the motion data and cardiac data. In some aspects, the instructions may further adapt the health monitor to calculate a second caloric burn rate for a second activity performed by the subject for a first time interval of a sequence of first time intervals, and determine whether the second caloric burn rate falls within at least one recommended range of values proscribed by a health entity for the duration of the first time interval. In some implementations, the instructions may further adapt the health monitor to record a first credit value for each first time interval that the second caloric burn rate falls within a first recommended range of the at least one recommended range for the duration of the first time interval, and record a second credit value for each first time interval that the second caloric burn rate falls within a second recommended range of the at least one recommended range for the duration of the first time interval.

The terms "sensor" or "monitor" as used herein may refer to a small electronic device configured to sense at least one parameter of a subject to which the sensor or monitor may be attached. A sensor may have limited processing power. A sensor may be configured to transmit data wirelessly or via a wired link to a second device. "Sensor" or "monitor" may also be used as shorthand when referring to a health monitor or intelligent health monitor, the intended meaning being apparent from the context.

The terms "intelligent sensor," "intelligent activity monitor," "activity monitor," or "intelligent monitor" as used herein may refer to a small electronic device configured to sense at least one parameter of a subject, and to process data representative of a detected activity. Such a device has greater processing power than a sensor, and may include at least one digital processor. An intelligent sensor may be configured to transmit and/or receive data wirelessly or via a wired link to and/or from a second device. "Health monitor" may also be used as shorthand when referring to an intelligent health monitor, the intended meaning being apparent from the context.

The term "digital processor" or "processor" as used herein may refer to at least one microcontroller, microprocessor, digital signal processor (DSP), application-specific integrated circuit (ASIC), or field-programmable gate array (FPGA). "Digital processor" may also be used to refer to any combination of the foregoing digital processing devices, including more than one of a particular device.

The terms "versatile sensor" or "versatile monitor" as used herein may refer to a small electronic device configured to sense at least one parameter of an entity, to process received data, and to flexibly participate in a small-area network. A versatile sensor may be configured to transmit data wirelessly or via a wired link to a plurality of devices.

The term "health monitor" as used herein may refer to a single intelligent sensor or a sensing system that may include at least one intelligent sensor. A health monitor may include one or more versatile sensors in some embodiments, and may include one or more sensors in some embodiments.

The foregoing and other aspects, embodiments, and features of the present teachings can be more fully understood from the following description in conjunction with the accompanying drawings.

BRIEF DESCRIPTION OF THE DRAWINGS

The skilled artisan will understand that the figures, described herein, are for illustration purposes only. It is to be understood that in some instances various aspects of the invention may be shown exaggerated or enlarged to facilitate an understanding of the invention. In the drawings, like reference characters generally refer to like features, functionally similar and/or structurally similar elements throughout the various figures. The drawings are not necessarily to scale, emphasis instead being placed upon illustrating the principles of the teachings. The drawings are not intended to limit the scope of the present teachings in any way.

FIG. 1A depicts examples of components that may be included in a health monitor, according to some embodiments;

FIG. 1B is a block diagram illustrating examples of selected electrical components that may be included in a health monitor, in some implementations;

FIGS. 1C-1F depict various embodiments of health monitors that are configured to be attached to a subject, and that include an LED display for communicating information to a user;

FIG. 1G depicts elements of a patch-type health monitor that includes activity sensing and heart-rate sensing, in some embodiments;

FIG. 2A is an illustrative example of a state diagram for low-energy operation of a health monitor, according to some embodiments;

FIG. 2B is an illustrative example of a state diagram for low-energy operation of a health monitor, according to some embodiments;

FIGS. 2C-2D depict illustrative examples of energy-conserving methods for operating a health monitor, in some implementations;

FIG. 2E illustrates a PQRST cardiac waveform, according to some embodiments;

FIG. 2F illustrates types of data that can be detected with a health monitor and fitness metrics that can be computed, according to some implementations;

FIG. 3 depicts an example architecture of a data processing system of a health monitor, according to some embodiments;

FIGS. 4A-4B depict illustrative examples of multi-axis accelerometer data and combined acceleration-derivative data for various types of activities;

FIGS. 5A-5C illustrate examples of membership functions that may be used in fuzzy-logic identification of different types of activities, according to some embodiments;

FIG. 6 depicts acts of a method 600 for processing activity data, according to some embodiments;

FIG. 7A illustrates acts associated with a sub-process for determining activity types and intensity levels, according to some embodiments;

FIG. 7B illustrates acts associated with a sub-process for determining health credits, according to some embodiments;

FIG. 7C illustrates acts associated with a sub-process for determining health credits, according to some embodiments;

FIG. 8A represents an example of a metabolic equivalents (METs) look-up table, according to some implementations;

FIG. 8B represents an example of a health-credit data stream that may be produced by a health monitor, according to some implementations;

FIG. 8C represents an example of compressed health credit data that may be stored on board a health monitor, according to some implementations;

FIG. 9A represents an example of health credit data that may be stored on board a health monitor in a temporary activity buffer, according to some implementations;

FIG. 9B represents examples of compressed health credit data and activity carry data that may be produced and stored on board a health monitor, according to some implementations; and

FIGS. 10A-10B depict illustrative examples of multi-axis accelerometer data and combined acceleration-derivative data for an activity with the health monitor attached to a subject at various locations.

The features and advantages of the present invention will become more apparent from the detailed description set forth below when taken in conjunction with the drawings.

DETAILED DESCRIPTION OF EXAMPLE EMBODIMENTS

I. Overview

As described above, the inventors have appreciated that conventional methods of using pedometers and activity monitors are primarily based on step counts, and that many of such conventional devices inaccurately record step counts during a day of use. For example, the inventors have observed that pedometers manufactured by different commercial entities have widely varying degrees of accuracy in reporting steps. Two activity monitors attached to the same subject can report a number of steps taken by the subject that differ as much as 25%, and number of calories burned that can differ by more than 50%.

With such variability among different activity monitors, the inventors have recognized that their use in a clinical setting as a diagnostic tool is compromised. For example, a physician prescribing an exercise regimen for an individual may not know with reasonable certainty whether the prescription is being fulfilled by the individual, or being filled in a way that the individual derives health benefits from the activity. Accordingly, exercise regimens designed for fitness conditioning or medical purposes (e.g., disease treatment or health recovery from an incident) may not be reliably formulated and/or monitored based upon step counts.

The inventors have recognized that activity monitors can be improved in terms of data acquisition and data analyses by computing health benefit levels or units, or "health credits" for activities performed by a user and detected by the activity monitor. Evaluation of health credits may be tied to recognizable health standards, and can be a more meaningful and universal evaluation of an activity than raw numbers relating to other parameters such as number of steps, distance traveled, calories burned, and duration of an activity. Health credits may be based in part upon parameters detected by conventional activity monitors, but the data may be processed further to better assess a quality of the activity performed.

The inventors have developed apparatus and methods that may be used to determine health credits of an activity performed by a subject. According to some embodiments, a health monitor system is configured to detect an activity performed by a subject, identify an activity type, and determine an intensity level of the performance of that activity. A health monitor may be configured to determine, at fine time resolution, continuous duration intervals for which the activity is performed, and compute a health credit amount based upon the continuous duration intervals, the intensity levels, and guidelines established by a health entity.

The inventors have also recognized that in some embodiments, the accuracy of calculations of health credits may be improved using cardiac data (e.g., heart-rate data from a cardiac sensor attached to a subject) in combination with motion data (e.g., motion data from an accelerometer supported by the subject) to compute caloric burn for various activities performed by the subject. Accordingly, embodiments of the apparatus may capture and analyze cardiac waveform data in addition to motion data. Additionally, the inventors have realized that a variety of different fitness metrics may be determined from cardiac data used alone or in combination with motion data.

Determining health benefit levels for performed activities and determining fitness metrics using the methods described herein can provide more accurate and useful diagnostic tools to evaluate exercise regimens and fitness levels of subjects. By evaluating physical activity in terms of health benefit, the individual or physician may have a more accurate representation of monitored exercise than step counts. Additionally, the individual or physician may readily determine whether the individual is receiving any health benefits from the exercise.

Various embodiments of apparatuses, methods, and systems for a health monitor are described in further detail in the following sections of the specification.

II. Example Apparatus

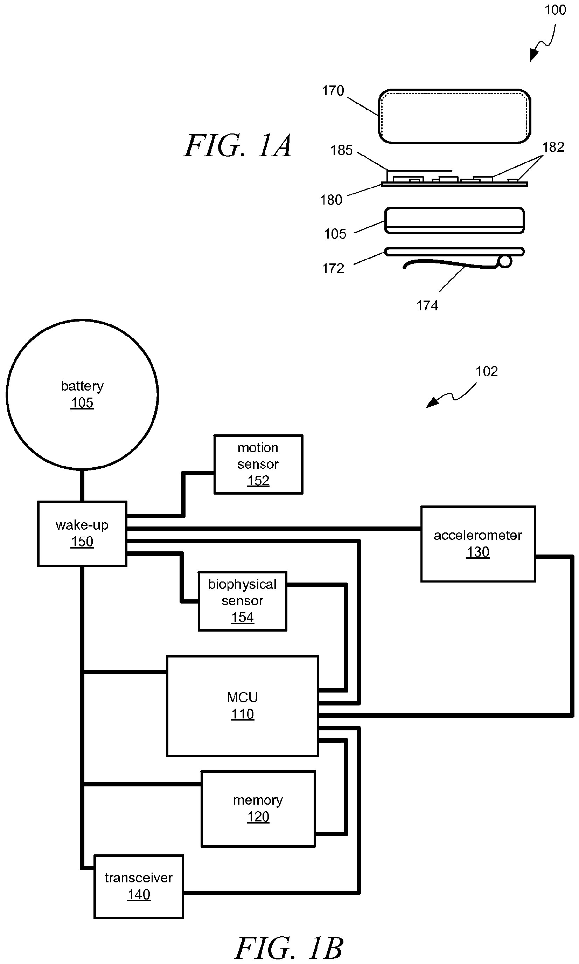

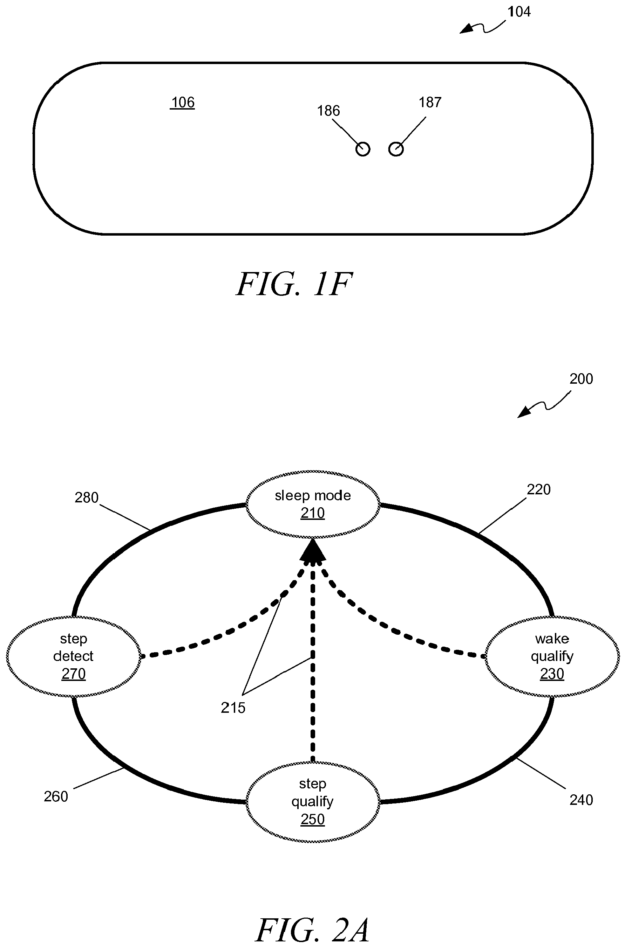

With reference to FIG. 1A, a health monitor 100 may comprise a small electronic device that can be attached to or supported by a subject, and that may be configured to identify a type of activity from among a plurality of different activity types that may be performed by the subject. Activity data (e.g., data including motion data and/or physiological data) produced by a health monitor during exercise performed by the subject may be processed on-board, in some embodiments, to determine "health credits" that quantitatively represent health benefits received from the activity. The health credits may be based at least upon activity duration, intensity level of the activity, and health standards established by a health entity. In some embodiments, health credits may further be based in part on activity type. In some implementations, the health monitor 100 may be configured to distinguish between different types of activities as well as detect non-human activities (*e.g.,* falsified activities such as strapping a health monitor to a wheel or fan), so that health credits are not awarded for such detected activities.

In some embodiments, a health monitor 100 may comprise an intelligent activity monitor, as depicted in the exploded view of FIG. 1A. A health monitor 100 may, for example, comprise an enclosure that includes a first cover 170 and second cover 172. The first and second covers may be formed from any suitable materials including, but not limited to, metals and plastics or combinations thereof. As one example, the first cover 170 may be a molded plastic and the second cover 172 a corrosion-resistant metal. The first and second covers may be fastened together by any suitable means and may form a water-tight seal enclosing a power source 105 and electronic circuitry 180 of the health monitor. A clip or strap 174 may be disposed on or attached to a surface of one of the covers so that the health monitor 100 may be attached to, or supported by, a subject or machine (*e.g.,* strapped to a wrist, ankle, or appendage, clipped to an article of clothing, strapped or clipped to a movable portion of a machine, such as an exercise machine or wheelchair.)

As shown, the electronic circuitry 180 may, for example, comprise a combination of circuit elements 182 disposed on a printed circuit board. In various embodiments, the circuit elements 182 may, for example, include a selected combination of integrated circuit (IC) chips, application-specific integrated circuit (ASIC) chips, at least one digital processor, micro-electrical-mechanical system (MEMS) devices, resistors, capacitors, inductors, diodes, light-emitting diodes, transistors, and/or conductive circuit traces, *etc.* A microcontroller or microprocessor may, for example, coordinate and manage operation of the intelligent monitor's electronic circuitry and process activity data, in some embodiments. In some embodiments, the electronic circuitry 180 may further include at least one radio-frequency (RF) antenna 185 for use in sending and receiving RF communication signals.

FIG. 1B depicts in further detail internal circuitry 102 that may be used in a health monitor 100, according to some embodiments. As shown, the health monitor's circuitry may, for example, comprise a source of power 105, *e.g.,* at least one battery or energy-scavenging chip and a wake-up and power-management circuit 150, that provide and manage power to an accelerometer 130, a digital processor 110, memory 120, and a transceiver 140. The processor 110 may be coupled to the wake-up circuit, the accelerometer, memory, and the transceiver. The processor may be configured to receive and process acceleration data from the accelerometer 130, to read and write data to memory 120, and to send and receive data from transceiver 140. The wake-up circuit 150 may be adapted to sense when the health monitor 100 is not in use, and in response, reduce power consumption of the internal circuitry 102, according to some embodiments. The wake-up circuit may be further adapted to sense when the health monitor 100 is placed in use, and in response, activate one or more elements of the internal circuitry 102.

In some embodiments, the processor 110 may, for example, comprise a low-power, 8-bit processor configured to draw low power in sleep-mode operation, and capable of operating at multiple millions of instructions per second (MIPS) when activated. One example of a suitable processor is the 8051F931 processor available from Silicon Laboratories Inc. of Austin, Texas. Another example of a processor is the nRF51822 processor available from Nordic Semiconductor of Oslo, Norway, though any other suitable processor or microprocessor may alternatively be employed in other embodiments. In some implementations, the processor 110 may support radio-frequency communications with other devices. A balun (*e.g.,* BAL-NRF02D3 available from ST Microelectronics of Geneva, Switzerland) may be used to match RF signals between an antenna and the processor, according to some embodiments.

The processor 110 may, for example, include various types of on-board memory (*e.g.,* flash memory, SRAM, and XRAM) for storing data and/or machine-readable instructions, and may be clocked by an internal oscillator or external oscillator. In some embodiments, the processor may, for example, be clocked by an internal high-frequency oscillator (*e.g.,* an oscillator operating at about 25 MHz or higher) when the processor is active and processing data, and alternatively clocked by a low-frequency oscillator (external or internal to the processor) when the processor is substantially inactive and in sleep mode. The clocking of the processor at low frequency may, for example, reduce power consumption by the processor during sleep mode. The low-frequency clocking may be at a frequency that is less than 50% of the high-frequency clocking in some embodiments, less than 20% of the high-frequency clocking in some embodiments, less than 10% of the high-frequency clocking in some embodiments, less than 5% of the high-frequency clocking in some embodiments, less than 2% of the high-frequency clocking in some embodiments, less than 1% of the high-frequency clocking in some embodiments, and yet less than 0.1% in some embodiments.

In various embodiments, the processor 110 may be configured to receive acceleration data from accelerometer 130 and process the received data according to preprogrammed machine-readable instructions that are loaded onto and execute on the processor. The processor 110 may, for example, be configured to receive analog and/or digital input data, and may include on-board analog-to-digital and digital-to-analog converters and on-board timers or clocks. According to some embodiments, the processor may also be configured to receive and analyze cardiac waveform data. In some embodiments, the processor may be further configured to receive power through wake-up and power management circuitry 150. The processor may, in some embodiments, cooperatively operate with or comprise a portion or all of power management circuitry 150, and facilitate in the activating and deactivating of one or more circuit elements within the health monitor.

In some embodiments, the processor 110 may be configured to be operable at a number of different clock frequencies. When operating at a low clock frequency, the processor will typically consume less power than when operating at a high clock frequency. In some embodiments, the processor may, for example, be configured to be in a "sleep" mode and operating at a low clock frequency when there is no motion of health monitor 100, and to be cycled through several operating states when motion of the health monitor 100 is detected. An example of how a processor may be cycled in such manner will be described below in reference to FIG. 2A. As one example, when in sleep mode, the processor may sample data at a rate less than 10 Hz and draw less than about 30 microamps.

In some embodiments, accelerometer 130 may, for example, comprise a multi-axis accelerometer configured to sense acceleration along at least two substantially orthogonal spatial directions. The accelerometer 130 may, for example, comprise a three-axis accelerometer based on micro-electro-mechanical systems (MEMS) technology. In some implementations, one or more single-axis accelerometers may additionally or alternatively be used. In some embodiments, the accelerometer 130 may be configured to provide one or more analog data-stream outputs (*e.g., X, Y, Z* data outputs corresponding to each axis of the accelerometer) that are each representative of a magnitude and direction of acceleration along a respective axis. One example of a suitable accelerometer is the Kionix model KXSC7 accelerometer available from Kionix Inc., Ithaca, New York. Another example of a suitable accelerometer is the LIS2DH accelerometer available from ST Microelectronics of Geneva, Switzerland. The accelerometer 130 may, for example, provide analog output data, that may later be converted to digital data, or may provide digital output data representative of acceleration values.

The accelerometer 130 may be characterized by several parameters. Among these parameters may, for example, be a sensitivity value and a sampling rate value. As examples, the accelerometer's analog sensitivity may be between about 100 millivolts (mV) per gravitational value (100 mV/G) and about 200 mV/G in some embodiments, between about 200 mV/G and about 400 mV/G in some embodiments, between about 400 mV/G and about 800 mV/G in some embodiments, and yet between about 800 mV/G and about 1600 mV/G in some embodiments. When configured to provide a digital output, the sampling rate of the accelerometer may, for example, be between about 10 samples per second per axis (10 S/sec-A) and about 20 S/sec-A in some embodiments, between about 20 S/sec-A and about 40 S/sec-A in some embodiments, between about 40 S/sec-A and about 80 S/sec-A in some embodiments, between about 80 S/sec-A and about 160 S/sec-A in some embodiments, between about 160 S/sec-A and about 320 S/sec-A in some embodiments, and yet between about 320 S/sec-A and about 640 S/sec-A in some embodiments. It will be appreciated that in some embodiments the higher sampling rates may improve the quality of the measured accelerations.

It will be appreciated that, in some embodiments, an accelerometer 130 may be combined with one or more analog-to-digital converters to provide digital output data representative of acceleration values at sampling rates described above. When digital output data is provided by an accelerometer, the accelerometer's sensitivity may be expressed in units of bits per gravitational constant (b/G). As examples, an accelerometer providing digital output data may have a sensitivity of more than about 2 b/G in some embodiments, more than about 4 b/G in some embodiments, more than about 6 b/G in some embodiments, more than about 8 b/G in some embodiments, more than about 10 b/G in some embodiments, more than about 12 b/G in some embodiments, or even higher values in some embodiments.

According to some embodiments, a health monitor 100 may include one or more sensors in addition to motion sensor 152 and accelerometer 130. For example, a health monitor may include at least one physiological sensor 154 (*e.g.,* cardiac sensor, temperature sensor, blood glucose sensor, blood oxygenation sensor, *etc.*) configured to sense at least one physiological parameter of a subject. Examples of physiological parameters that may be sensed in some embodiments include, but are not limited to, cardiac waveform, skin temperature, core temperature, respiration rate, plethysmography waveform, EKG waveform, blood oxygenation level, blood glucose level, hydration, blood pressure, *etc.* One illustrative example of a physiological sensor comprises the AD8232 ECG chip available from Analog Devices, Inc. of Norwood, Massachusetts. Such a chip may be combined with electrodes arranged to contact the skin of a subject. A physiological sensor 154 may be disposed in a same package with a health monitor in some implementations, or may be formed as a separate monitor to be attached to the subject at a separate location and wirelessly, or via a wired link, transmit data to the health monitor according to a predetermined communication protocol.

In some embodiments, a health monitor 100 may include memory 120 that is external to and accessible to the processor 110. The memory 120 may be any one of or combination of the following types of memory: RAM, SRAM, DRAM, ROM, flash memory. The memory 120 may, for example, be used to store and/or buffer raw data from accelerometer 130 and/or physiological sensor 154, machine-readable instructions for processor 110, program data used by the processor for processing accelerometer data and/or physiological data, and/or activity data representative of an activity. In some embodiments, the memory 120 may additionally or alternatively be used to store diagnostic information about the health of the health monitor, *e.g.,* battery life, error status, *etc.*, and/or physical parameters about the device, *e.g.,* memory size, gravitational sensitivity, weight, battery model, processor speed, version of operating software, user interface requirements, *etc.* In some embodiments, the memory may also be used to store information pertinent to a user, *e.g.,* user weight, height, gender, age, training goals, specific workout plans, activity-specific data for a user that may be used to identify an activity performed by the user or process data representative of an identified activity. According to some embodiments, the memory 120 may store tables of metabolic equivalents (METs), calibration values, and health guideline data that is used to determine health benefit levels for various activities.

In some embodiments, the memory 120 may additionally or alternatively be used to store data structures and/or code received from an external device, *e.g.,* via a wired or wireless link. The data structures and/or code may, for example, be used to update one or more data processing applications used by the health monitor. For example, one type of data structure may be data representative of an acitivity data pattern that may be used to identify a specific type of activity not previously recognized by the health monitor, *e.g.,* a new activity or an activity that is specific to an individual user of the health monitor. As another example, a data structure may comprise a membership function, described below, defined for a new activity or redefined for an identifiable activity. According to some embodiments, the data structure may, for example, include one or more sample accelerometer traces and physiological data obtained during performance of the activity and/or may comprise identification data (*e.g.,* membership functions) resulting from the processing of the accelerometer traces that may be used in an algorithm executed by the health monitor 100 to identify the activity. Further, in some embodiments, the memory 120 may be used to store updates and/or replacements to algorithms executed by the health monitor. The stored data structures and algorithms may, for example, be used to reprogram and/or expand the functionality of the health monitor 100 to identify new activities or activities not previously recognized by the health monitor and/or improve the accuracy or confidence of results calculated for identified activities.

In some embodiments, the memory 120 may also be used to store calibration and/or conversion data that is used by the processor 110 to characterize detected activities. Calibration data may, for example, be used to improve the accuracy of detected activity parameters (*e.g.,* stride length, speed), and/or improve the accuracy of fitness metrics computed from detected activities. Conversion data may, for example, be used to convert a detected activity into an amount of expended human energy, *e.g.,* calories burned, metabolic equivalents, *etc.*

According to some embodiments, a health monitor 100 may include a transceiver 140 and/or one or more data communication ports (*e.g.,* a USB port, an RF communication port, a Bluetooth port) for communicating data between the health monitor and one or more external devices such as a computer, tablet, cell phone, portable communication device, data processor, a sensor, another intelligent sensor, or a versatile sensor, any of which may be configured to communicate with other similar devices in a network such as the world-wide web or a local area network. A health monitor 100 may, for example, be configured to communicate via the transceiver 140 through a wired or wireless port to any device or combination or devices selected from the following list: a personal computer, laptop computer, tablet computer, PDA, a watch, an MP3 player, an iPod, a mobile phone, a medical device such as a blood glucose meter, blood pressure monitor, or InR meter, an electronic interactive gaming apparatus, intelligent training equipment, and an automobile system. Data retrieved from the memory 120 or to be stored in memory 120 may, for example, be communicated between the health monitor 100 and an external device via the transceiver 140. In some embodiments, data transmitted from the health monitor 100 may be configured for routing to a data service device adapted to process data received from a health monitor.

In some embodiments, power for the internal electronics of a health monitor 100 may be provided by at least one battery 105 and managed by a wake-up and power-management circuit 150. The battery may be small, *e.g,* a button-cell type, and may, for example, comprise one or more lithium-type batteries that may be rechargeable or replaceable. As just one example, a single lithium coin or button-cell, 3-volt battery having a capacity of about 230 mAh may be used (model CR2032 available from Renata SA of Itingen, Switzerland). Another embodiment of a health monitor may include one or more model CR1616 batteries, though any suitable type of battery may alternatively be used in various embodiments. In some embodiments, a health monitor may include power-generation or energy-harvesting hardware (*e.g.,* a piezo-electric material or electric generator configured to convert mechanical motion into electric current, a solar cell, an RF or thermal converter). Power that is generated on board may be stored in a battery or charge storage component such as a super capacitor. In some implementations, generated electrical current may be provided to a storage component via a diode bridge. One example of a suitable energy harvesting device is a microenergy cell MEC225 available from Infinite Power Solutions, Inc. of Littleton, Colorado. In some embodiments, power generation components may be used in combination with a rechargeable battery as a source of power for a health monitor 100. A voltage regulator chip (*e.g.,* TPS78001 available from Texas Instruments of Dallas, Texas) may be used to condition power from at least one power source before delivering the power to components of a health monitor, according to some embodiments.

In some implementations, wake-up and power-management circuitry 150 may include a motion sensor 152 that, in combination with the wake-up and power-management circuitry 150, identifies when a health monitor 100 is being moved in a manner that may be representative of an activity to be monitored. The wake-up and power-management circuitry 150 may, for example, comprise logic and control circuitry to enable, disable, reduce and/or increase power to various circuit elements shown in FIG. 1B. Logic and control circuitry for the wake-up and power-management circuitry may, for example, comprise machine-readable instructions and utilized hardware of the processor 110, or may comprise machine-readable instructions and utilized hardware of an application specific integrated circuit.

In some embodiments, the motion sensor 152 may comprise one or more force sensitive switches, *e.g.,* a piezo element configured to generate an electric signal representative of an amount of acceleration that a health monitor experiences. In other embodiments, the motion sensor 152 may additionally or alternatively comprise one or more contact switches that close a circuit, or open a circuit, when the health monitor is subjected to an acceleration, *e.g.,* a "ball-in-tube" switch. Wake-up may, for example, be initiated when a frequency of switch closures exceeds a pre-selected value. In other embodiments, the sensor 152 may additionally or alternatively comprise one or more force-sensitive contact switches that close only when a health monitor undergoes acceleration in excess of a pre-selected value.

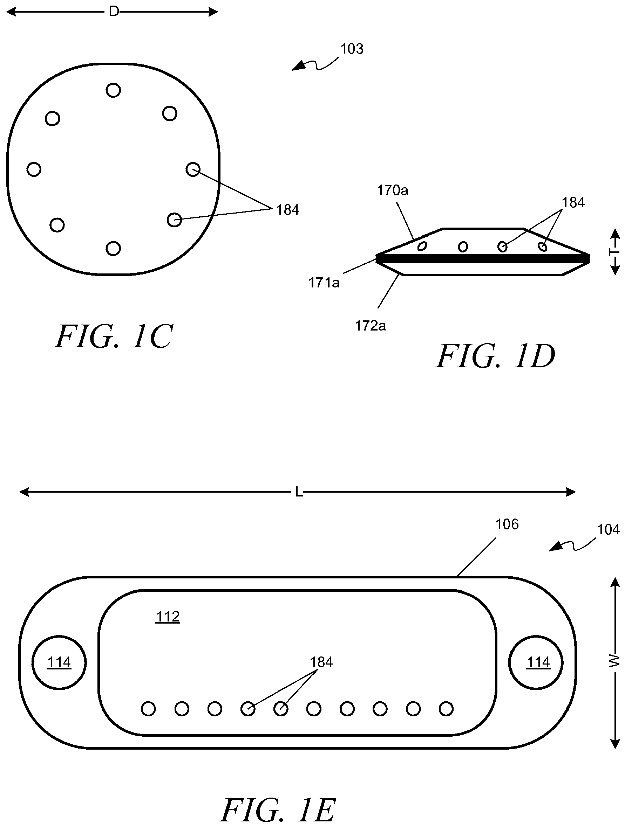

FIGS. 1C-1D illustrate an example embodiment of a health monitor 103 that includes light-emitting diodes (LEDs) 184 for communicating information to a user. The illustrations show a packaged device in plan view (FIG. 1C) and elevation view (FIG. 1D). A health monitor may be substantially round, rectangular, square, elliptical, or be in the form of a multi-sided polygon. A health monitor may have a largest dimension D that is between about 5 mm and about 40 mm in some embodiments, and a thickness T that is between about 1 mm and about 10 mm in some embodiments. The size of the health monitor 103 may be largely determined by its power source, *e.g.,* the size of its battery. A health monitor may have a form factor that substantially matches a form of the power source. For example, a health monitor may have a shape similar to the shape of a power source, and have a volume that is up to 20% larger than the volume of the power source in some embodiments, up to 50% larger than the volume of the power source in some embodiments, up to 100% larger than the volume of the power source in some embodiments, and yet up to 200% larger than the volume of the power source in some embodiments. The LEDs may be disposed in a line, a circle, an ellipse, or any other shape. A first cover 170a and second cover 172a may snap or be screwed together, so as to form a seal with a polymer gasket 171a. In some embodiments, a clip or strap may include the polymer gasket 171a, so that the device could be easily changed from a clip-on device to a strap-on device. In some implementations, a clip or strap may removably attach to the first or second covers. In yet other embodiments, a clip or strap may cradle the health monitor.

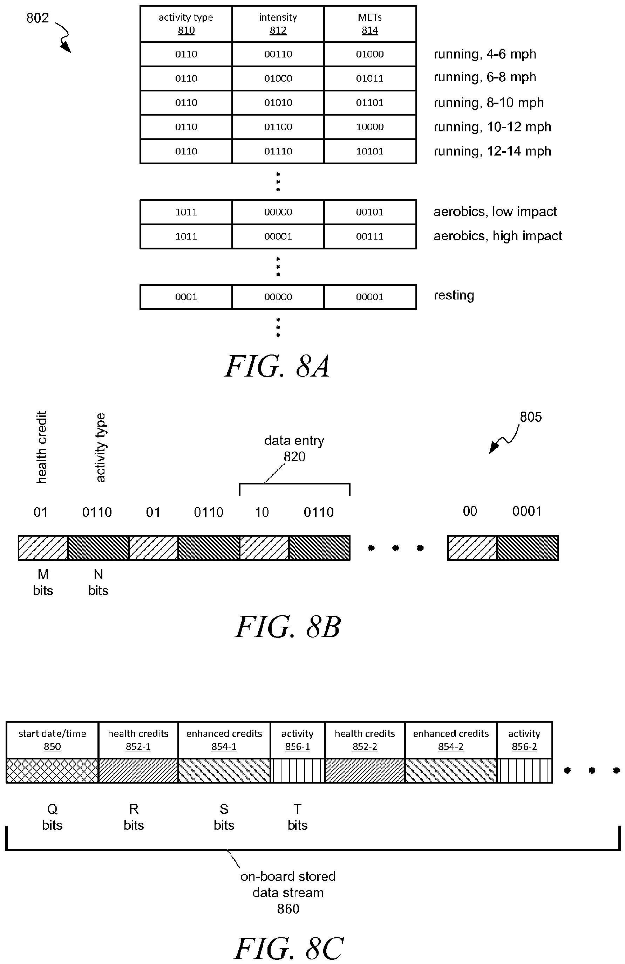

According to some embodiments, a health monitor 103 may be configured to recognize one or more tapping sequences and/or motion gestures (*e.g.,* moving the device in a figure 8 pattern, a circle pattern, a back-and-forth linear pattern), and activate the LEDs to communicate information responsive to the detected tapping sequence or gesture. A tapping sequence or gesture may correspond to a particular information query, to which the health monitor may respond with the appropriate information. According to one embodiment, the health monitor may be tapped in a particular manner, and in response activate a number of LEDs to indicate that a user has reached an approximate percentage of an activity goal (*e.g.,* 8 of 10 LEDs to signal approximately 80%). Information about progress toward one or more activity goals can be communicated by the device (*e.g.,* walked 30% of a goal of 3 miles, ran 60% of a goal of 8 miles, swam 90% of a goal of 60 laps, achieved 70% of creditable health-beneficial activity for the day, achieved 50% of a recommended number of health credits for a week, *etc.*) The LEDs may also be used to communicate other information responsive to particular tapping sequences or gestures, *e.g.,* battery life, pace comparison (ahead of, or behind, best pace for an activity), heart rate, calories burned, *etc._*Although LEDs may be used to communicate information to a user, a small liquid-crystal display may be used in some embodiments instead of, or in addition to, LEDs.

FIGS. 1E-1G illustrate example embodiments of health monitors 104, 160 according to some embodiments. A health monitor 104 may be formed in the shape of a band having a central compartment 112 and fastening features 114. The central compartment 112 may, for example, be attached to a flexible band 106 that can be strapped around a limb of a subject or attached to a subject in any suitable manner (*e.g.,* strapped around an ankle region, a wrist, or an arm). The central compartment may house electronic components 180, 185, 182 (see FIG. 1A) of the health monitor. In some embodiments, the health monitor 104 may include one or more LEDs 184 for conveying information to a user, as described above. In some embodiments, the central compartment may be flexible (*e.g.,* formed of a polymer or elastomer), and electronic components of the monitor may be mounted on a flexible substrate having flexible interconnects. In some embodiments, the central compartment 112 and band 106 are semi-rigid, and the fastening features 114 attach to flexible bands that are used to strap the device to a limb. The fastening features may be holes, button snaps, a buckle, clasps, or any other suitable fastening feature. The health monitor 104 may, for example, have a length L between about 10 mm and about 250 mm or longer, a width W between about 5 mm and about 30 mm, and a thickness T between about 1 mm, and about 10 mm. In some embodiments, the health monitor 104 may have a curved profile along its length so that is may better conform to the contour of a limb.

According to some embodiments, a health monitor 104 may include at least one light source 186 and at least one photodetector 187. The at least one light source and photodetector may be used, for example, for sensing one or more physiological parameters of a subject, *e.g.,* blood oxygenation level, plethysmography waveforms, blood glucose level, *etc.* In some embodiments, the light source 186 may comprise a high-brightness infrared (IR) photodiode and a shorter wavelength photodiode. In recent years, progress in indium-gallium-nitride LED technology has yielded devices with both lowered junction voltage and increased radiated intensity. Using InGaN technology, and applying power management techniques described below, may provide a health monitor capable of measuring heart rate that can run for multiple months on small silver-oxide batteries, having a form-factor such as that of a typical bandage or wristwatch.

The photodetector 187 may be any suitable photodetector, and may be mounted to detect light from the light source that is scattered or reflected from the subject.

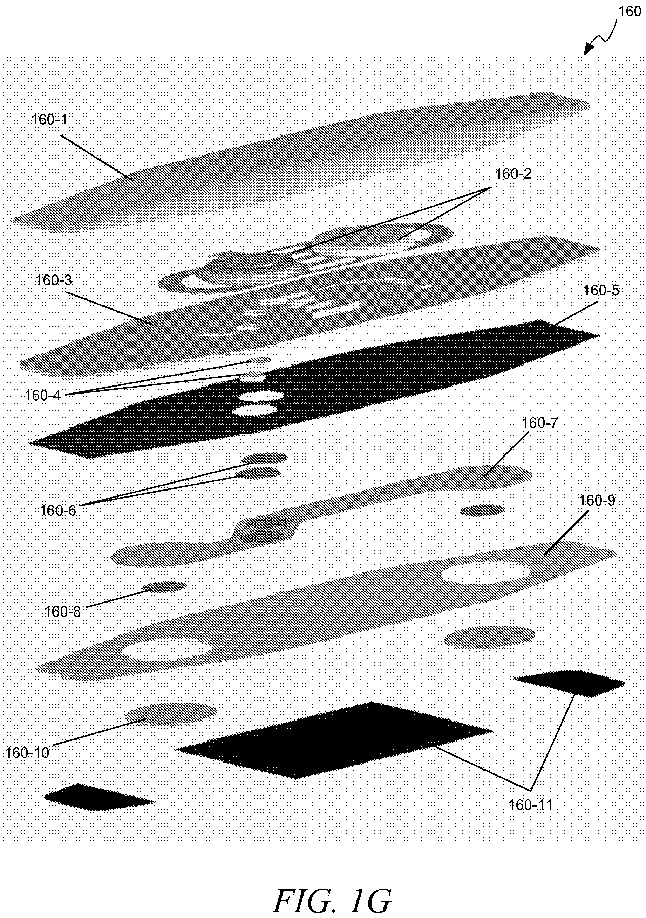

FIG. 1G depicts a patch-type health monitor 160, according to some embodiments. A patch-type health monitor may be flexible, thin, and in the form of an adhesive patch, and may include some disposable parts in some implementations. For example, a patch-type health monitor may comprise a flexible cover 160-1, which may be formed of silicone. The health monitor 160 may include a first flexible printed circuit board (PCB) 160-3 to which at least one monitor (accelerometer and/or cardiac sensor) may be connected. Power may be provided by one or more batteries (*e.g.,* coin cell batteries such as CR1616 batteries). A patch-type health monitor may include multi-component electrodes for electrically contacting a subject to sense cardiac waveforms. For example, the electrodes may comprise first conductive pads 160-4 (*e.g.,* copper pads) having a diameter between approximately 2 mm and approximately 6 mm and a thickness between approximately 0.1 mm and approximately 1 mm, according to some embodiments. The first conductive pads may adhere to a second flexible PCB 160-7, which may have a thickness less than 1 mm. Conductive tape 160-6 may be used to adhere the first conductive pads 160-4 to the second flexible PCB 160-7. In some embodiments, second conductive pads 160-8 (*e.g.,* silver chloride pads) may contact the second flexible PCB and further contact adhesive hydro-gel pads 160-10 (*e.g.,* adhesive material 9880 available from 3M Corporation of St. Paul, Minnesota) that are arranged to contact the subject at separated locations. In some implementations, a first adhesive layer 160-5 (*e.g.,* LSE adhesive 96042 available from 3M Corporation of St. Paul, Minnesota) may be used to adhere the first flexible PCB layer 160-3 to the second flexible PCB layer 160-7 and an intervening hydrocolloid layer 160-9 (*e.g.,* hydrocolloid 9943 available from 3M Corporation of St. Paul, Minnesota). In some embodiments, a second biocompatible adhesive layer 160-11 (*e.g.,* adhesive 2475P available from 3M Corporation of St. Paul, Minnesota) may be used to adhere the patch-type health monitor 160 to a subject. Cardiac signals may be conveyed to an ECG chip located on the first flexible PCB 160-3 by circuit paths extending through the hydro-gel pads 160-10, second conductive pads 160-8, second flexible PCB 160-7, conductive tape 160-6, and first conductive pads 160-4.

In some embodiments, a patch-type health monitor 160 may have a thickness between approximately 1 mm and approximately 4 mm and a length between approximately 30 mm and approximately 100 mm. The width of the monitor may be between approximately 10 mm and approximately 30 mm. According to some embodiments, the patch-type monitor may be configured to be adhered to the torso of a subject near the heart, *e.g.,* in the vicinity of the second to fourth rib of a subject. The device may be waterproof, and configured to adhere to a subject for a period of one or more days. In some implementations, the device's biocompatible adhesive may allow adhesion for a period of time greater than four days or more. In some embodiments, the two hydro-gel pads 160-10 may be separated a distance between approximately 15 mm and approximately 50 mm. In some implementations, the separation distance may be between 20 mm and 30 mm.

According to some aspects, a the hydro-gel pads 160-10 and second biocompatible adhesive layer 160-11 may be replaceable when the health monitor is removed from a subject and re-adhered. For example, a subject may remove the health monitor 160 at any time and re-adhere it at a later time. Although the health monitor may be configured to be worn continuously, a subject may wish to remove it daily in some cases, weekly in some cases, monthly in some cases, or at any suitable interval. After removal, the hydro-gel pads 160-10 and second biocompatible adhesive layer 160-11 may be removed from the health monitor and replaced with fresh pads and adhesive.

In some implementations, a cardiac monitor of a health monitor 160, as depicted in FIG. 1G, may be configured to capture cardiac waveform data continuously in some modes of operation and intermittently in some modes of operation. The types of information that may be determined by a health monitor 160 from cardiac waveforms may include heart rate, inter-beat interval (IBI), heart rate variability (HRV), PQRST waveform profile, and respiration rate.

Although the health monitors are depicted as a single packaged device in FIGS. 1E-1G, in some embodiments a health monitor may comprise two or more separate sensors attached to or supported by a subject that are configured to communicate over a small-area network or body local-area network (body LAN). One or more of the sensors may be configured as a versatile sensor, as described in U.S. Patent Application No. 13/840098 titled "Versatile Sensors with Data Fusion Functionality," filed March 15, 2013, and incorporated herein by reference in its entirety. In some embodiments, additional sensors may detect other physiological data and communicate representative data to health monitor 104.

Referring now to FIGS. 2A-2B, in some embodiments, the wake-up and power-management circuitry 150 for a health monitor may be configured to cycle the device through a plurality of operational states, as depicted in the drawings. The operational state to which the health monitor is cycled may, for example, depend upon motion detected by the wake-up and power-management circuitry, or data received from another sensor in a body LAN. Some of the operational states may, for example, be power-conserving, low-power, or no-power states.

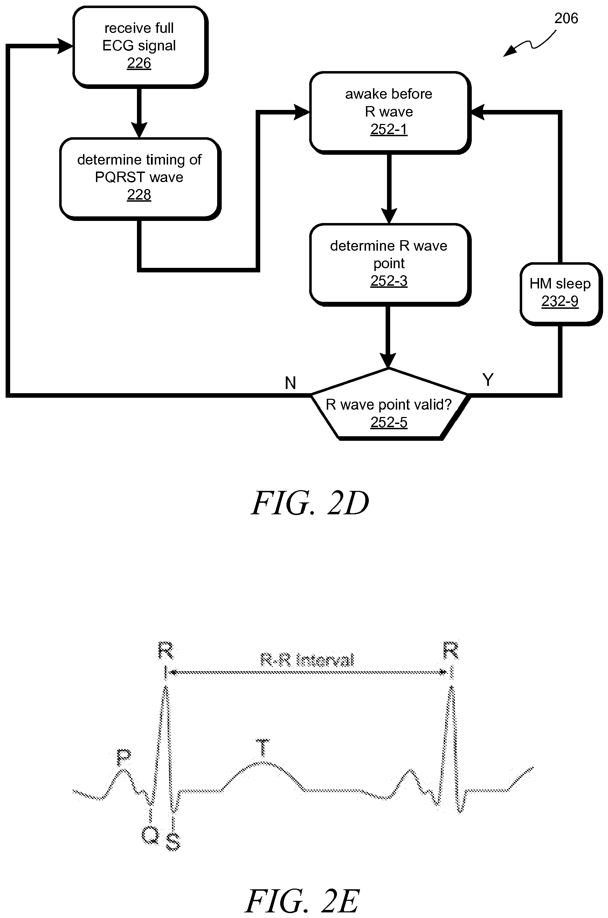

According to some embodiments, there may be one low-power or no-power state 210 and one or more powered operational states 230, 250, 270. The powered operational states may, for example, include various power-conserving states. As illustrated, a health monitor may move from any one state to any other state along paths 220, 240, 260, 280, 215. In some embodiments, there may be paths in addition to or in lieu of those shown in FIGS. 2A-2B, *e.g.,* directly from step detect state 270 to wake qualify state 230.

In some embodiments, when a subject is inactive (*e.g.,* the health monitor is exhibiting no motion or motion less than a first pre-selected limit or threshold), the monitor may operate in a sleep mode 210. In some embodiments, sleep mode may consume no power. In other implementations, however, sleep mode may consume low power, *e.g.,* draw about 1 microamp or less. The low power may, for example, be provided to the motion sensor 152 for detecting a motion of a health monitor 100. In some embodiments, no power is provided to any combination of or all of the motion sensor 152, physiological sensor 154, the processor 110, memory 120, and transceiver 140 while in sleep mode. According to some embodiments, when sufficient motion has been detected via the motion sensor 152, the health monitor may be moved to a wake qualify state 230.

In some embodiments, sufficient motion for moving the health monitor out of the sleep state 210 may be detected via motion sensor 152 according to an amplitude and/or frequency of a signal from the motion sensor. For example, when the motion sensor comprises one or more piezo-electric elements, a signal greater than a pre-defined signal value may be used to identify sufficient motion of the health monitor and move the monitor to wake qualify state 230. In another example, the motion sensor 152 may comprise one or more contact switches, and a pre-defined number of switch openings, or closings, per predefined time interval may be used to identify sufficient motion of the health monitor and move the health monitor to wake qualify state 230.

In some embodiments, when in the wake qualify state 230, low levels of current (*e.g.,* less than about 30 microamps) may be consumed by a health monitor 100 in the system. Power may, for example, be provided to the accelerometer and to the systems processor so that data from the accelerometer may be processed while in the wake qualify state 230. Power may, in some embodiments, also be provided to memory 120 while in the wake qualify state. In some implementations, power may be provided to one or more physiological sensors in a wake qualify state.

In some embodiments, when in the wake qualify state 230, the system may be clocked at a low rate to conserve power. For example, the processor 110 may be clocked at a low frequency and/or the accelerometer sampled at a low frequency (*e.g.,* less than about 10 Hz) to obtain and process data from the accelerometer. The data may, for example, be processed to determine whether a predefined second threshold has been exceeded. In some embodiments, the second threshold may comprise a pre-defined amount of force or acceleration that a health monitor 100 is subjected to, and may include a further parameter, *e.g.,* a frequency of occurrence of measured values exceeding the pre-defined amount of acceleration, a heart rate. When the second threshold is crossed, the health monitor may, for example, be moved to an activity qualify or step qualify state 250. If the second threshold is not crossed within a pre-defined period of time, the health monitor may, for example, return to sleep mode 210.

In some embodiments, when in a step qualify state 250, more power is provided to the accelerometer 130 and/or processor 110, so that a greater amount of data processing of motion data can occur. The accelerometer and/or processor may, for example, may be operated at a higher clock frequency so that a greater amount of data may be obtained from the accelerometer and processed by the processor for a given time interval, as compared with the wake qualify state 230. The current consumed by a health monitor in step qualify mode may be on the order of a few hundred microamps, *e.g.* between about 100 microamps and about 500 microamps in some embodiments. The data-collection rate may, for example, be increased to a normal operating rate in step qualify mode 250. The accelerometer data may, for example, be sampled at several hundred Hertz (*e.g.,* 256 Hz) or higher values. In some implementations, a greater amount of physiological data may be obtained and processed in a step qualify state.

In some embodiments, when in the step qualify mode 250, a health monitor 100 may analyze detected acceleration data to determine whether the data is representative of an activity that may be a recognizable activity for the monitor 110, *e.g.,* walking, running, swimming, jumping rope, *etc.* In some embodiments, if it is determined by the processor that the activity may be recognizable, the health monitor may be moved to a step detect state 270. In some embodiments, if it is determined that there is insufficient activity in a pre-defined amount of time to be recognizable by the processor, then the health monitor may return to wake qualify mode 230.

According to some embodiments, when the step detect mode 270 is executed, a health monitor may be placed in full operation. In this mode, power may, for example, be provided to transceiver 140, in addition to the other operational components so that communications with an external device or additional sensors may be carried out. In some embodiments, normal operating clock frequencies and sampling rates may be used for operating the accelerometer, one or more physiological sensors, and processor, so that activity detection and full data processing may be carried out. Physiological sensors may, or may not, be fully powered in a step detect mode 270. Current consumption in step detect mode 270 may, for example, be several hundred microamps, *e.g.,* between about 200 microamps and about 600 microamps.

In some embodiments, each operational state other than sleep mode 210 may include a provision for returning a health monitor directly to sleep mode, *e.g.,* along state paths 215 as indicated in FIG. 2A. For example, each operational state 230, 250, 270 may be configured to return a health monitor to sleep mode 210 if there is a termination of incoming data or of processed data parameters associated with incoming data. In some embodiments, a health monitor may additionally or alternatively be configured to return to sleep mode upon a system glitch or crash, *e.g.,* a freezing of the processor. A return to sleep mode may, for example, be used to reset a health monitor.

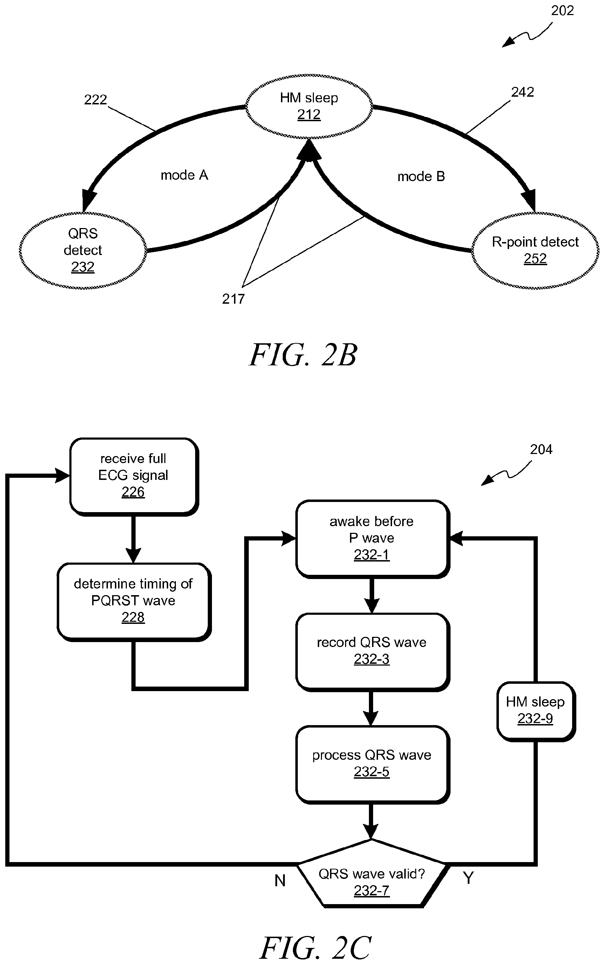

According to some implementations, power conservation for the health monitor may additionally or alternatively be based on cardiac data, as depicted in FIG. 2B. Power conservation methods based on cardiac data may run in parallel with or in combination with power conservation methods based on motion data. As just one illustrative example, motion data may be analyzed by a system processor to determine that a subject is in an inactive state (*e.g.,* sitting, lying, riding in a vehicle, *etc.*). A health monitor may then determine that at least power to a motion detector and physiological sensor may be reduced. In some embodiments, a motion sensor may enter a sleep mode 210, and a cardiac sensor configured to sense a subject's cardiac waveform may also enter a sleep mode 212, as shown in FIGS. 2A-2B. To extend battery life, the cardiac sensor may cycle through one or more low-power operational modes from a sleep state, as depicted in FIG. 2B while the subject is inactive.

For example and referring to FIG. 2B, in a first mode (mode A), a cardiac sensor may "sleep" (sleep state 212) for a period of time between each heartbeat of a subject and "awake" in time to capture a waveform (QRS detect state 232) of a heart beat (*e.g.,* a QRS complex) for subsequent analysis. The QRS waveform may, for example, be analyzed for arrhythmia, heart rate variability, and/or respiration rate, according to some implementations. In some embodiments, respiration rate may be determined from an envelope of the R-wave over multiple cardiac cycles. In a first mode, full waveform capturing circuitry may be utilized to sample and analyze the cardiac waveform during only the QRS complex.

In a second mode of operation (mode B), a heart monitor may sleep for a period of time between each heartbeat of a subject and awake in time only to determine a point or timing of an R wave (R detect state 252). In some implementations, the cardiac signal may be fed to a comparator configured to detect a threshold crossing or change in slope of the R wave. The comparator may require less power to operate than circuitry needed to capture and analyze a portion of the cardiac waveform.

In a third mode of operation (not shown in FIG. 2B), a heart monitor may operate continuously to capture a full cardiac waveform for multiple beats. A continuous mode of operation may be executed periodically to ascertain the timing of P waves, in some embodiments, and determine an interval of sleep between heartbeats. In some implementations, a continuous mode of operation may be executed when a subject becomes active, or may be executed when a subject's activity is found to be moderate and/or vigorous. In some implementations, a user may command continuous monitoring of a cardiac waveform irrespective of the user's activity by a tapping sequence on the patch or motion sensor that can be detected by the motion sensor, processed, and recognized by the health monitor's processor.

In further detail, FIGS. 2C-2D are illustrative examples of methods of capturing and processing cardiac signals which a health monitor may be configured to implement. For reference, an example of a cardiac waveform is depicted in FIG. 2E, which illustrates the P, Q, R, S, and T portions of a cardiac cycle.

According to some embodiments and referring to FIG. 2C, a method 204 of cardiac-waveform-based power conservation implemented on a health monitor may comprise receiving 226 an ECG signal for a period of time and analyzing the data to determine 228 a timing of PQRST waveforms for a subject. According to some embodiments, a health monitor may awaken 232-1 a heart monitor before a P-wave, record 232-3 a QRS complex, and process 232-5 the QRS complex to obtain cardiac data (*e.g.,* arrhythmia, heart rate variability, and/or respiration rate). The health monitor may awaken the heart monitor while keeping the motion sensor in a sleep mode, according to some embodiments. The heart monitor may be activated between approximately 1 ms and approximately 100 ms before a P-wave, according to some embodiments. In other embodiments, the heart monitor may be activated later or sooner. The health monitor may determine 232-7 whether the recorded QRS waveform was valid (*e.g.,* not clipped or missing a portion due to incorrect time gating). If the QRS wave is valid, the health monitor may enter a sleep mode 232-9 during which power to the heart monitor is reduced or shut off until a next P-wave. If it is determined that the QRS waveform is not valid, the health monitor may power up the heart monitor and associated electronics to receive 226 a full ECG signal for a period of time so that an appropriate sleep or awake interval can be determined.

In some implementations and referring to FIG. 2D, a method 206 of cardiac-waveform-based power conservation implemented by a health monitor may comprise receiving 226 an ECG signal for a period of time and analyzing the data to determine 228 a timing of PQRST waveforms for a subject. A health monitor may awaken 252-1 the heart monitor before an R wave (*e.g.,* between approximately 1 ms and approximately 100 ms before the R-wave, according to some embodiments, though other times may be used in other embodiments). In this mode, a comparator may be awakened and used to process an input cardiac signal rather than using A/D circuitry and digital processing of a QRS complex. By detecting a threshold crossing point or change from positive to negative slope of the R-wave, the health monitor's processor may determine 252-3 an R-R interval between heartbeats. This data may be used to evaluate inter-beat interval (heart rate) and/or heart-rate variability. The health monitor may assess 252-5 whether the determined R-wave point is valid (*e.g.,* occurring within an expected window of time). If it is determined that the determined R-wave point is valid, the heart monitor may enter a sleep mode 232-9. If it is determined that the R-wave point is not valid, the health monitor may power up the heart monitor and associated electronics to receive 226 a full ECG signal for a period of time.

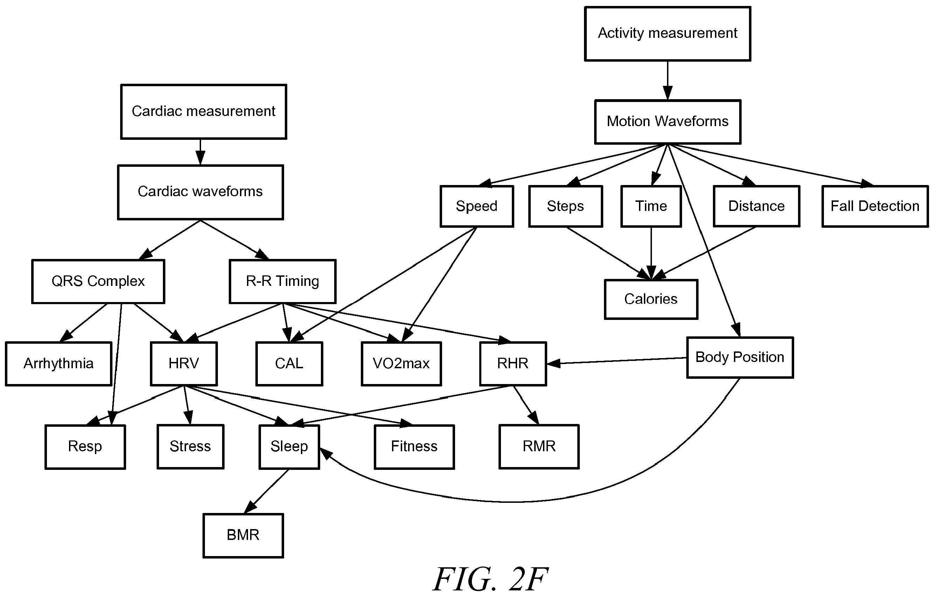

FIG. 2F depicts types of data that may be obtained from a motion sensor and cardiac waveform sensor of a health monitor, according to some embodiments. For example, a motion sensor (*e.g.,* accelerometer) may be used for various activity measurements to obtain motion waveforms corresponding to a type of activity performed by a subject, as will be described in further detail below. Analogously, a cardiac sensor may be used to obtain cardiac waveforms for a subject during different levels of activity and during inactive periods. In some embodiments, data from the different sensor types may be used separately in some instances and may be combined in other instances to determine different fitness metrics for an individual, some of which are depicted in the drawing.

As just one example, motion waveforms may be analyzed to determine a body position of a subject to which a health monitor is attached. The motion waveforms may indicate that the subject is in a prone position. Analysis of cardiac waveforms or of R-R interval data obtained during the same period may indicate, for example, a lower than normal heart rate, which may be determined by the health monitor to be a resting heart rate (RHR). In some embodiments, the combined motion and cardiac data may indicate that the subject is asleep, and may be used, for example, to determine a basal metabolic rate (BMR) for the subject. Further, heart rate variability (HRV) data along with motion data from a motion sensor may be used to assess a quality of the subject's sleep. For example, low heart rate variability for a subject may indicate increased stress for the subject. In some embodiments, low HRV may be determined from a ratio of low-frequency (LF) and high-frequency (HF) spectral powers of a cardiac waveform. Spectral powers of a cardiac waveform may be determined from a Fourier transform or FFT of cardiac cycles. LF spectral power may be in a range between about 0 and 150 Hz, and HF spectral power may be in a range between about 150 Hz and about 400 Hz. An increased LF/HF ratio for a subject over an average value during a sleeping state may indicate an increased level of stress for the subject and a reduced quality of sleep. Additionally or alternatively, excessive motion during sleep may indicate a reduced quality of sleep. Other fitness metrics that may be determined from cardiac and motion data are described below.

III. Data Processing

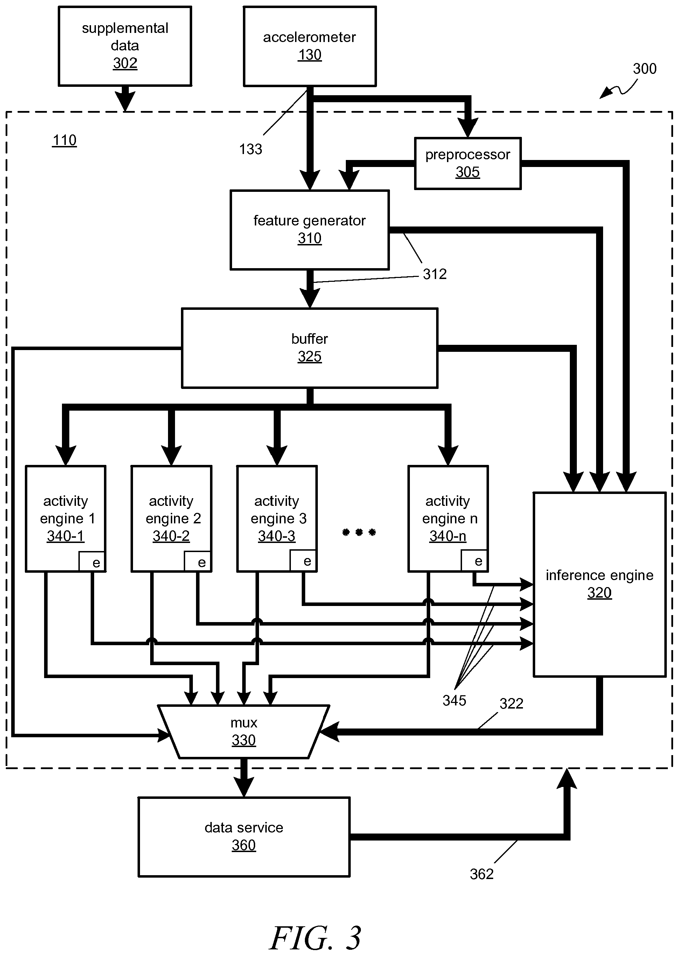

This section provides an overview of data handling and data processing paradigms that may be implemented with various embodiments of a health monitor. In some implementations, the processing of activity data may be carried out on one or more processors of a health monitor. With reference to the block diagram of FIG. 3, an illustrative example of a data handling architecture 300 for a health monitor is shown, according to some embodiments. In some embodiments, the data handling architecture may be implemented at least in part on a processor 110 that is specially adapted with appropriate machine-readable instructions.

Data handling architecture 300 for a health monitor may comprise a data preprocessor 305, a feature generator 310, a buffer 325, an inference engine 320, and at least one activity engine 340-1, according to some embodiments. In some embodiments, data handling architecture may further include a multiplexor 330 and a data service 360. In some implementations, any of the preprocessor 305, feature generator 310, inference engine 320, multiplexor 330, and activity engines 340-1 - 340-n may be embodied in whole or in part as machine-readable instructions operable on processor 110 that, when executed, adapt the processor to perform a respective functionality as described below, or as implemented in alternative embodiments. Further, any of the preprocessor 305, feature generator 310, inference engine 320, multiplexor 330, and activity engines 340-1 - 340-n may additionally or alternatively be embodied in whole or in part as hardware configured to perform a respective functionality or a portion of the respective functionality.

It should be appreciated that other embodiments may include fewer, additional, or different data-processing elements than those shown in FIG. 3. For example, a data post-processor may be added before or after data service 360. Moreover, it should be appreciated that, in some embodiments, one or more depicted elements may be combined into a single unit providing equivalent functionality of both units depicted separately. One or more of the components depicted in FIG. 3 may be implemented in hardware (*e.g.,* as field programmable gate arrays, digital signal processor, and/or an application-specific integrated circuit) and/or a combination of hardware and machine-readable instructions (*e.g.,* software) that may be executed on a digital processor.

As illustrated in FIG. 3, in some embodiments, an accelerometer 130 may be configured to output a motion data stream 133 of values representative of acceleration detected by the accelerometer along at least one direction of motion. The motion data stream 133 may, for example, comprise acceleration values representative of acceleration measured along respective *X*-, *Y*-, and *Z*-axes of motion defined at the accelerometer 130. In some embodiments, the data stream 133 of acceleration values may be provided to both a feature generator 310 and a preprocessor 305. In some implementations, supplemental data 302 (e.g., physiological data such as any one or combination of cardiac data, respiratory data, blood oxygenation or blood glucose data, *etc.*) received from one or more physiological sensors, may be provided to the processor 110, and may be provided to the feature generator 310 and/or preprocessor 305. According to some embodiments, the preprocessor 305 may, for example, pre-process at least the motion data stream 133 from the accelerometer to produce a stream of acceleration-derivative values that is provided to the feature generator 310. In some embodiments, acceleration-derivative values may not be computed. Any of the data may be filtered, for example, to reduce noise components or select specific frequency components for analysis.

In some implementations, supplemental data 302 may be provided to one or more processing devices of the processor 110. For example, supplemental data 302 may be provided to any one or combination of preprocessor 305, feature generator 310, and inference engine 320. In some implementations, the preprocessor 305 may, for example, pre-process a plethysmography waveform or cardiac waveform to generate data characteristic of a subject's heart beat, which may be provided to the feature generator 310. Supplemental data 302 may also be provided to buffer 325, in some embodiments. Supplemental data 302 may be analyzed to provide additional information about an activity (*e.g.,* the processor 110 may be configured to analyze cardiac data to determine a pulse rate, for example, from which an intensity level at which an activity was performed may be estimated) and/or to verify a type of activity performed (*e.g.,* distinguishing between riding an exercise cycle, riding a bicycle on flat terrain, or riding a bicycle up a hill).

In certain embodiments, the feature generator 310 may process the received data stream 133 of acceleration values, received supplemental data, and received preprocessed data (*e.g.,* a stream of acceleration-derivative values) to produce one or more characteristic features 312 that may be provided to an inference engine 320 and optionally to a buffer 325. The characteristic features may, for example, be used by the inference engine 320 to identify a type of activity sensed by the sensors from among a plurality of types of activities. In some embodiments, upon the identification of an activity type, the inference engine 320 may provide a control signal 322 to a multiplexor 330 to route the characteristic features 312 to an appropriate activity engine 340-1, 340-2, ..., 340-n for further data processing. Each activity engine may, for example, be configured to process received characteristic features according to activity-specific algorithms (*e.g.,* algorithms for running, walking, swimming, biking, *etc.*) to calculate one or more parameters descriptive of the activity. Illustrative examples of the one or more parameters include, but are not limited to, a measure of intensity or pace of the activity, a calculation of energy expended during the activity, an estimation of distance traveled during the activity, and a duration of the activity.

In some embodiments, the buffer 325 may be used to temporarily retain data representative of an activity while inference engine 320 identifies an activity. For example, once an activity has been identified, data may be routed from the buffer to the appropriate activity engine or engines. Such temporary buffering of data prevents loss of activity data during initial identification of an activity or transitions from one activity to another.

According to some embodiments, once an activity has been identified, data from the feature generator 310, which may include raw accelerometer data, may be routed to an appropriate activity engine 340-m (m corresponding to the value of a selected engine 1, 2, 3, ... n). Each of the activity-specific data processing engines may, for example, use any instance or combination of acceleration-derivative data $D_n$, one or more accelerometer trace data, physiological data, and characteristic feature data to determine a value of one or more parameters associated with the activity (*e.g.,* speed, distance, intensity, number of steps, *etc.*). In some embodiments, physiological data may be used in combination with motion data to determine an intensity of the activity and/or other parameters associated with an activity.

In some embodiments, a selected activity engine, inference engine, or post-processing element (not shown) may additionally or alternatively determine an energy expenditure (*e.g.,* calorie burning rate) for the activity. According to some embodiments, the energy expenditure may, for example, be determined at least in part from a look-up table of metabolic equivalents (METs) for the activity. The look-up table may, for example, comprise a list of metabolic equivalents where each entry may be associated with one or more activity intensity parameters, *e.g.,* step rate, speed, heart rate, *etc.* A METs look-up table for each activity that may be performed by a human may, for example, be stored in memory 120 of the health monitor. In some embodiments, look-up tables for METs may be user-specific, *e.g.,* specific to a user's sex, weight, age, and/or height. In some embodiments, over the course of an activity session, a health monitor may determine and record caloric burn rates as a function of time and may also compute a total number of calories burned as a function of time of the activity. In some embodiments, a health monitor may additionally or alternatively compute and/or store other data, e.g., activity type, maximum speed, average speed, distance traveled, number of steps, maximum caloric burn rate, average caloric burn rate, time of day *etc.*

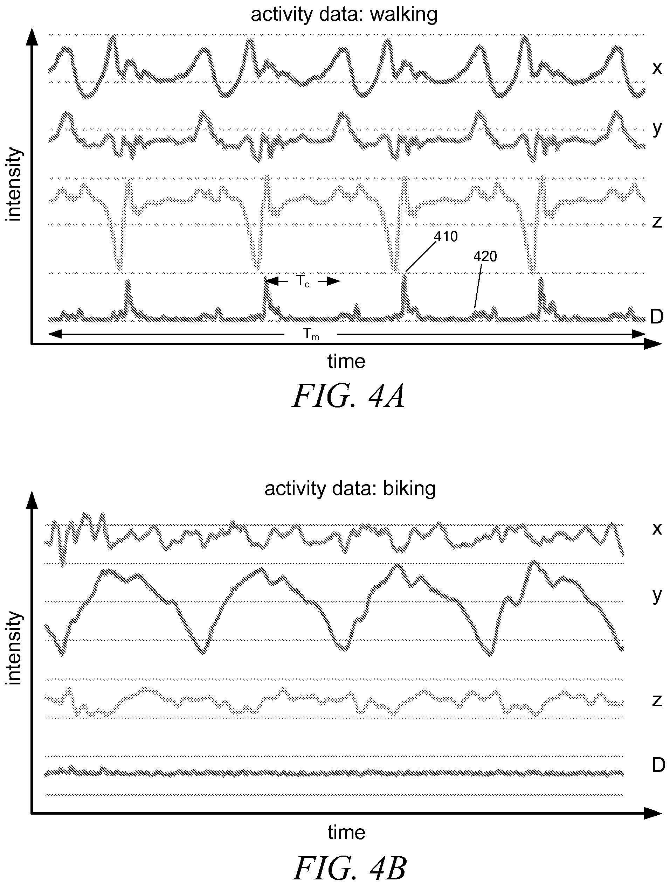

As one example of processing motion data and referring to FIG. 4A, an inference engine 320 may identify an activity represented by the data in the figure as walking. Accordingly, the multiplexor may, for example, be configured to forward acceleration-derivative data $D_n$ to a walking activity engine 340-1. Walking activity engine 340-1 may, for example, be configured to determine a distance $T_c$ in the data between a large peak 410, which corresponds to a heel strike, and a successive smaller peak 420, which corresponds to a toe-off in a step. The distance $T_c$ may, for example, represent the contact time of the foot with the ground, from which a walking speed can be determined. An example of a method of determining walking speed from foot contact time is disclosed in U.S. Patent No. 4,578,769, which is hereby incorporated by reference in its entirety.

In various embodiments, the preprocessor 305 and feature generator 310 may preprocess raw data from the accelerometer and/or received supplemental data 302 in any suitable manner, *e.g.,* filter the data, compute derivative values, compress the data, generate data sets, packetize the data, *etc.* Some examples of data preprocessing by the preprocessor 305 and feature generator 310 are described in U.S. Patent Application No. 13/840098 titled "Versatile Sensors with Data Fusion Functionality," referenced and incorporated by reference above. In various embodiments, the preprocessor and feature generator process received raw data to reduce the processing burden on the inference engine 320 and activity engines 340.

According to some implementations, inference engine 320 may additionally or alternatively be configured to identify activities as non-recognizable (*e.g.,* activities that may be performed by a subject but for which the health monitor is not programmed to recognize), as well as identify activities that are not performed by a human (*e.g.,* non-human activities that might be performed by a machine or animal). The inference engine 320 may identify activities based upon data received from one or more of the preprocessor 305, feature generator 310, and activity engines 340. In some embodiments, raw acceleration data may additionally or alternatively be provided to inference engine 320 for identification of detected activities. In some embodiments, a health monitor may classify activities into two or more classifications, *e.g.,* (i) recognizable activities, (ii) non-recognizable activities that are likely performed by the subject, and (iii) non-human or non-animate activities (*e.g.,* activities performed by a machine or animal). A health monitor may be configured to subsequently present an activity identified as non-recognizable to a user for identification by the user, in some embodiments, and the health monitor may be modified for subsequent recognition of the activity (*e.g.,* the health monitor learns or constructs one or more membership functions encompassing characteristic features for the activity).

In some embodiments, an inference engine may receive a set of characteristic features 312 (representative of a non-recognizable activity) that cannot be identified as corresponding to any one of a plurality of activity types recognizable by the inference engine. In this case, the data-handling architecture may, for example, be configured to provide the characteristic feature set data 312 directly to data service 360 for subsequent analysis and determination of a type of activity associated with the feature set (*e.g.,* identifying the activity by user-assistance, or by comparing with on-line libraries of identified activities having similar feature sets). The feature set data 312 may be routed by the multiplexor 330 from the buffer 325 to data service 360. Further, the data-handling architecture may be additionally or alternatively configured to receive machine-readable instructions and reconfiguration data 362 back from data service 360 suitable to reconfigure inference engine 320 and/or add or modify an activity engine 340 to subsequently identify a type of activity corresponding to the previously non-recognizable feature set. In this manner, activity recognition by the health monitor may, for example, be upgraded or reconfigured at any time.

In some embodiments, a non-recognizable activity may be reported to a user for subsequent identification by the user at a time well after completion of the activity. For example, a non-recognized activity may be reported when the user is reviewing a record of monitored activities via a computer. The user may, for example, be notified that a non-recognizable activity occurred at a specific date and time and for a duration, and then the user may be queried to identify the activity. In some embodiments, the user may then identify the activity, which will in turn associate a set of characteristic features 312 with the previously non-recognized activity. According to some embodiments, a list of possible activities having a similar caloric burn rate may be presented to a user, so that the user may select an activity from the list. The list may comprise activities listed in the "2011 Compendium of Physical Activities: A Second Update of Codes and MET Values," by Barbara E. Ainsworth et al., published by the American College of Sports Medicine, the entire contents of which are incorporated herein by reference. The upgrading of the inference engine 320 and/or activity engine 340 may, for example, comprise transmitting new data structures and/or code to the processor 110 for use in recognizing the new activity.

In some implementations, an inference engine 320 may be configured to recognize one or more activities that are not performed by a human. Such non-human activities might be associated with mechanical motion of a machine, for example. One example of such an activity might be cyclic motion of a fan, wherein an health monitor may be strapped to the blade of a fan. Another example might be motion of an automobile, motorized vehicle, or motorized ride (*e.g.,* riding in a vehicle over rough terrain, a carnival ride, riding on a train). Another example might be motion of a bicycle wheel, *e.g.,* monitor attached to spokes of a wheel. Other examples of activities not performed by a human might be motion in a clothes drying or clothes washing machine. Another example may include walking or running motion of a dog or horse. In some cases, the activities not performed by a human may, for example, be recognized to prevent erroneous crediting of physical activity to a subject, *e.g.,* in connection with health insurance incentive programs or prescribed physical exercise. The inference engine may recognize non-human activities as having characteristics not capable of being achieved by a human, *e.g.,* precise repetitive motion or excessive acceleration values.

According to some embodiments, the inference engine 320 is configured to further receive error signal indications 345 from each activity engine 340-1 ... 340-n. An error signal may, for example, represent a confidence level of an activity processed by an activity engine. For example, a first activity engine 340-1 may be an activity engine for walking, and a second engine 340-2 an engine for running. When the user is walking, for example, a step cadence may be below a threshold criterion for running, and an output error signal from the running activity engine 340-2 might be high or a confidence level may be low. The output error signals may, for example, be used by inference engine 320 to aid in identifying an activity and/or transitions between activities. In some instances, all activity engines may substantially simultaneously process feature data, whereas in other instances only one activity engine or a selected number of activity engines may be selected to process feature data. According to some embodiments, the presence of a large error signal for a currently identified activity may, for example, cause inference engine 320 to re-identify an activity for newly received data.

In some embodiments, parameters characteristic of an activity including any data computed by an activity engine 340-m may be provided to and handled by data service 360 for subsequent presentation to the user, on-board storage, on board analysis, and/or storage in a remote storage device. Additional data (*e.g.,* date, time, and duration of activity) may be provided to the data service 360. The data may be formatted or packetized by the data service in any suitable format, and may be formatted to include header information. The data service 360 may, for example, comprise an on-board data store, *e.g.,* memory 120, and a transceiver 140 for transmitting the data to a remote device, such as a computer. In some implementations, data service 360 may comprise a temporary buffer that is sized to hold activity data for a time interval between about 5 minutes and about 30 minutes or longer periods. In some embodiments, data service may additionally or alternatively include an application in operation on a remote computer or a remote server configured to receive data from the health monitor and record and/or further process the received data. In some implementations, data 362 from the data service 360 may be retrieved by the processor 110 for further processing, *e.g.,* to determine health benefit levels from the recorded data.

In some embodiments, a health monitor 100 may additionally or alternatively be configured to store in memory user goals (*e.g.,* number of steps per day, distance traveled, an exercise duration for a specific activity, a number of health credits for a period of time, *etc*.). The processor 110 may be configured to determine a progress toward the user's goal or goals based on output from the activity engines, according to some embodiments. The health monitor may, for example, be further configured to provide an audible, visible, or tactile indication to the user to indicate a progress toward goals and/or indicate when a goal is reached. For example, a health monitor may flash LEDs, beep or vibrate when a user has reached a goal of awarded health credits for an interval of a day.

Because a health monitor may include an accelerometer and components for processing motion data, in some embodiments, a health monitor 100 may additionally be configured to recognize specific motion gestures that a user may execute (*e.g.,* shaking the health monitor, moving it in a circle with the hand, spinning the health monitor). A health monitor may, for example, include one or more activity engines adapted to recognize such gestures. The recognized gestures may, for example, be used as an interface method for executing specific functions on the health monitor (*e.g.,* power up, power down, clear data, set a goal, display progress toward one or more goals, *etc*.).

IV. Identification of Activity Types

Identification of activity types, according to some embodiments, will be described in further detail. In some embodiments, data generated by an accelerometer 130 and/or one or more physiological sensors may be processed to produce characteristic features $f_n$ that are provided to the inference engine 320 for activity type identification. The inference engine 320 may, for example, be configured to receive processed data, and in some cases raw data, and further process the received data to identify and/or classify a detected activity. In some embodiments, an activity that is identified may be one activity from among a plurality of different activity types that are performed by a human and that the health monitor is programmed to recognize.

According to some embodiments, the inference engine 320 may classify activities into different categories. The categories may include, for example, non-human activities, recognizable human activities, and non-recognizable human activities. The classification may occur as the inference engine attempts to identify an activity type. According to some embodiments, data for each classification may be handled differently to determine health credits for a subject, as described further below.

In some embodiments, the inference engine 320 may process received acivity data to identify an activity type in any suitable manner. In some implementations, the inference engine 320 may identify an activity based on acceleration-derivative data $D_n$ only (as described in U.S. Patent Application No. 13/840098 titled "Versatile Sensors with Data Fusion Functionality," referenced above), or a combination of raw acceleration-derivative data and physiological data and/or a limited set of characteristic features. In some embodiments, the inference engine 320 may identify an activity using one or more raw accelerometer traces and/or characteristic features generated from the one or more raw traces. In some cases, raw data or acceleration-derivative data may be used in combination, or in combination with physiological data, to identify or classify an activity. Further, in some embodiments, the inference engine may additionally or alternatively qualify the activity data (*e.g.,* identify a level of quality of the recognized activity's data or identify an aspect of the recognized activity such as a location of the health monitor on the subject) using any combination of acceleration-derivative data $D_n$, raw accelerometer data, physiological data, and related characteristic features.

According to some implementations, the inference engine 320 may employ one or more identification algorithms to identify or distinguish activities. For example, in some embodiments, the inference engine 320 may employ a pattern recognition algorithm to recognize an activity based upon a waveform defined by acceleration-derivative data $D_n$ and/or one or more raw accelerometer traces. In some embodiments, the inference engine 320 may additionally or alternatively employ fuzzy logic to identify an activity based upon a number of values in characteristic feature sets $F_n = \{f_1, f_2, ... f_n\}$. The inference engine 320 may thus, in some embodiments, employ a combination of pattern recognition and fuzzy logic to identify an activity. In some embodiments, for fuzzy logic recognition, membership functions specific to different activities may be defined and downloaded to the health monitor. It should be appreciated that other "recognition" algorithms or combinations of such algorithms may additionally or alternatively be used.

As just one example of activity recognition, data received by inference engine 320 that has a predetermined number of values falling within a membership function range may be identified by inference engine as an activity associated with that membership function. For some implementations, fuzzy logic may be suitable for recognizing a large variety of different activities without placing a heavy data-processing burden on processor 110. For example, fuzzy logic may, in some embodiments, require only determining whether a plurality of characteristic features from feature data $F_n$ fall within certain ranges of values. In other embodiments, fuzzy logic may additionally or alternatively evaluate a cost factor for each candidate activity and identify an activity based upon an evaluation of the cost factor.

For purposes of understanding only, and without limiting the invention, one example of activity identification is described with reference to FIGS. 4A - 4B. FIG. 4A represents three traces (*x, y, z*: top three) of raw acceleration data from a first type of activity (walking in this example). The lower trace D in FIG. 4A represents acceleration-derivative data computed from the upper traces. FIG. 4B represents corresponding traces of data obtained from a second type of activity (biking in this example). For the data shown in FIGS. 4A-4B a health monitor 100 was supported below the subject's shin.

As can be seen from the traces of FIGS. 4A-4B, there are a number of differences in the traces. The differences include maximum and minimum values of acceleration, periodicity of the traces, number and shapes of peaks in the traces, width of peaks, and distances between peaks, among other things. The differences may, for example, be captured in characteristic feature sets $F_n$ for each trace.

Although FIGS. 4A-4B depict only motion data derived from an accelerometer, it will be appreciated that additional data from one or more physiological sensors may be processed in a similar manner to generate feature sets for physiological data. A health monitor may process the physiological data in combination with the activity data to enhance information about a subject or activity performed by the subject. For example, the physiological data may provide an indication of an intensity level of an activity.

Continuing with the above example, in some embodiments, a characteristic feature set $F_n$ for each trace within a measurement interval $T_m$ may be constructed with the following entries:

$$F_n = \{\text{max value of trace (max); min value of trace (min); number of peaks with a width less than } m_1 \text{ samples } (N_p); \text{ number of valleys with a width less than } m_2 \text{ samples } (N_v); \text{ average rate of change of the trace at half-maximum of peaks } (R_{1/2}); \text{ average distance between peaks } (\Delta P)\}.$$

It should be appreciated that a wide variety of characteristic features may be generated and used to identify the different activities. As can be understood from this example, the differences in the traces of FIGS. 4A-4B may, for example, be captured as numerical differences in the characteristic feature sets. In some embodiments, the inference engine 320 may then distinguish between the activities based upon such numerical differences, *e.g.,* by comparing numerical values for various features against corresponding values for recognizable activity types. A judicious choice of values to include in feature sets may, in some embodiments, reduce the computational burden on inference engine 320 and enable rapid identification of different types of activities.

According to some embodiments, a membership function for each of the activities may, for example, be defined as follows:

$$M_{activity} = \begin{cases} \left(\text{max}_{avg} - \partial\text{max}\right) \leq \text{max} \leq \left(\text{max}_{avg} + \partial\text{max}\right); \\ \left(\text{min}_{avg} - \partial\text{min}\right) \leq \text{min} \leq \left(\text{max}_{avg} + \partial\text{max}\right); \\ \left(N_{p,avg} - \partial N_p\right) \leq N_p \leq \left(N_{p,avg} + \partial N_p\right); \\ \left(N_{v,avg} - \partial N_v\right) \leq N_v \leq \left(N_{v,avg} + \partial N_v\right); \\ \left(R_{\frac{1}{2},avg} - \partial R_{\frac{1}{2}}\right) \leq R_{\frac{1}{2}} \leq \left(R_{\frac{1}{2},avg} + \partial R_{\frac{1}{2}}\right); \\ \left(\Delta P_{avg} - \partial\Delta P\right) \leq \Delta P \leq \left(R\Delta P_{avg} + \partial\Delta P\right) \end{cases} \tag{1}$$

where the subscript "*avg*" designates an average or expected value, and the quantities $\pm\partial[]$ identify a pre-defined range within which a measured value would be considered to qualify as belonging to the membership function. In some embodiments, when characteristic features are received by an inference engine 320, for which all values qualify as belonging to the membership function, then the detected activity may be identified by the inference engine. The inference engine may then, for example, route the data from the feature generator, using multiplexor 330 to an appropriate activity engine for further analysis.

In some implementations, by sampling a large number of trials for each activity, variations in each of the characteristic feature values may be observed and statistics regarding the variations may be determined. The statistical results may, for example, be used to help construct membership functions for fuzzy-logic activity identification. For example, it may be observed that a maximum value of acceleration-derivative trace $D$ for running has a 2-sigma variation of 5 measurement units. A membership function for running may, for example, include the specification $\{(120 - 5) \leq \max$ value of $D \leq (120 + 5)\}$, where 120 measurement units is determined to be an average of the maximum value for trace $D$ for the running activity.

In some instances, there may be partial overlap of membership functions. For example, one or more ranges for characteristic features in one membership function may overlap or be coincident with corresponding ranges in a second membership function, which may or may not belong to the same activity type. Even though there may be partial overlap of membership functions, in some implementations, an activity type may be identified based on evaluating a plurality of characteristic features and their locations within membership functions for the activity types. For example, each activity may receive a score (*e.g.,* a value between 0 and 1) for each feature that falls within a membership function for that activity. In some embodiments, after scores have been tallied for each activity, the one receiving the highest score may be selected as the identified activity.

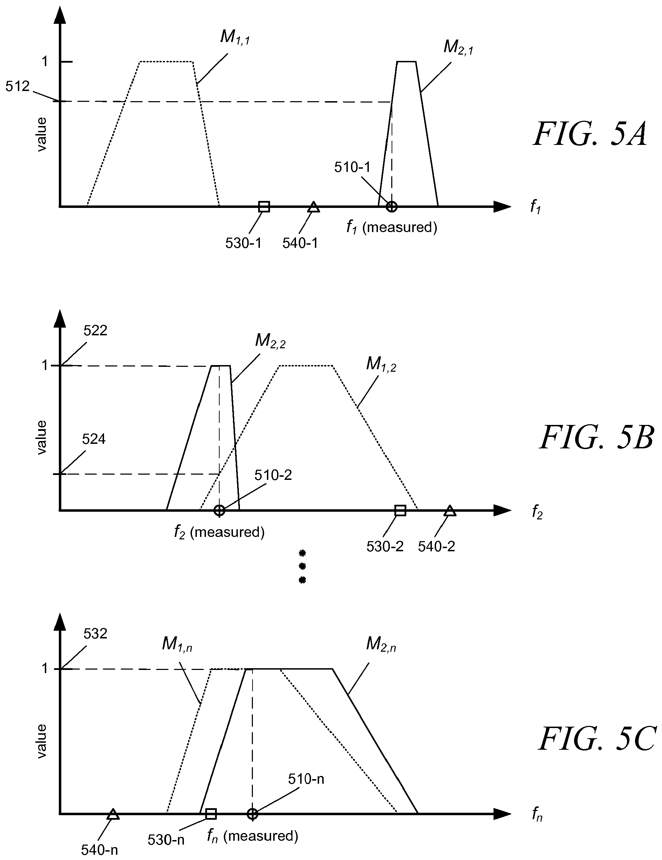

In some embodiments, scores based on the described membership functions may be all-or-nothing, *e.g.,* either a feature $f_n$ is measured and determined to be within the bounds of its corresponding membership function and contribute a score, or may be outside the bounds and contribute nothing. Other embodiments may additionally or alternatively employ membership functions such as those as depicted in FIGS. 5A-5C. The graphs depict membership functions $M_{i,j}$ that have been constructed for n characteristic features (denoted by the "*j*" subscript) and for two activities (denoted by the "*i*" subscript). The membership functions $M_{i,j}$ may, for example, be constructed from statistical analysis of many measurements, as described above. Though the membership functions are shown as trapezoidal, they may take any suitable shape, *e.g.,* round top, semi-circular, semi-ellipse, Gaussian, parabolic, distributions representative of measured statistical values, multi-modal distributions, *etc.*

In some implementations, when a characteristic feature is determined, *e.g.,* $f_1$ 510-1, a corresponding value for each activity's membership function for that feature may be determined. For the case shown in FIG. 5A, for example, the second activity's membership function $M_{2,1}$ contributes a value 512 where the first activity's membership function contributes no value. The values may be normalized, as depicted in the graphs. For a second measured feature 510-2 shown in FIG. 5B, both membership functions may, for example, contribute different values 522, 524. In this case, the membership functions overlap. For another measured feature 510-n shown in FIG. 5C, both membership functions may, for example, contribute identical values.

In some embodiments, a cost factor $C_i$ may be computed for each candidate activity (denoted by the "*i*" subscript) based upon detected feature values 510-1, 510-2, ... 510-n and the predetermined membership functions $M_{i,j}$ according to the following relation:

$$C_i = \frac{\sum_{j=1}^{n} W_{ij} M_{i,j}(f_j)}{\sum_{j=1}^{n} W_{ij}} \qquad (2)$$

where $W_{ij}$ represents a weighting factor for the $j^{th}$ feature of the $i^{th}$ activity. The weighting factor may, for example, be selected to emphasize some features and de-emphasize other features for purposes of identifying an activity. In some embodiments, an activity with the highest cost factor $C_i$ may be selected as the identified activity type. For example, with reference to FIGS. 5A-5C and considering only the membership functions shown and the measured feature characteristics 510-1, 510-2, ... 510-n, depicted as open circles, the second activity $M_2$ would be selected as the identified activity, according to some embodiments.

For EQ. 2, if the membership functions $M_{i,j}$ are each normalized, as depicted in FIGS. 5A-5B, the value of the cost function will range between 0 and 1. Multiplying the cost function by 100 can express a degree of matching as a percent confidence level $PC_i$.

$$PC_i = 100 \times C_i \qquad (3)$$

For example, a feature set that has each feature value falling under the peaks of the membership functions for a particular activity will yield a 100% confidence in the match and identification of the activity type.

In some cases, membership functions may overlap entirely, such that it would not be possible for the inference engine 320 to identify an activity between the two membership functions. This situation might occur, for example, when a new membership function is added to the inference engine 320 for recognition of a new activity not previously recognizable by the inference engine. For example, if the health monitor 100 is configured to identify biking and is later updated to identify elliptical training activity, a new membership function for identifying elliptical training may entirely overlap with the pre-defined membership function for biking since the two activities are similar.

In some embodiments, membership functions and characteristic features used by the inference engine 320 may be expandable, so that additional characteristic features and associated membership functions can be added to the system for the activities recognizable by the health monitor. The additional characteristic features and revised membership functions may, for example, be added to distinguish two activities that previously had substantially overlapping membership functions. The addition and updating of membership functions and features can be accomplished, for example, via communication between a health monitor with an external device, *e.g.,* a personal computer or computer connected to the internet.

Through the use of membership functions and/or pattern recognition, a health monitor may identify activity types. In some implementations, a health monitor may additionally classify detected activities into several categories. The categories may include, for example: (i) recognizable activities, (ii) non-recognizable activities that are likely performed by the subject, and (iii) non-human or non-animate activities (*e.g.,* activities performed by a machine). Recognizable activities would correspond to activity types (*e.g.,* running, walking, biking, swimming, *etc.*) that the health monitor has been programmed to recognize (*e.g.,* the monitor includes at least membership functions for these activity types). In various embodiments, activities falling within this first classification can be analyzed to identify a specific activity type, and would be candidate activities that may be further evaluated by the health monitor to determine a level of health benefit or health credit for the subject based on the identified activity. For recognizable activities, activity type may be determined by finding a high confidence of matching of characteristic features to membership functions for a particular activity. In some embodiments, the level of confidence may be expressed as a percentage of a maximum value obtainable for a best match.

Activities that fall with the second classification (non-recognizable activities that are likely performed by the subject) may or may not be further evaluated by the health monitor to determine a level of health benefit for the activity. In some embodiments, a health monitor may be configured to admit or reject a non-recognizable activity for health-benefit analysis based upon the activities similarity with a recognizable activity. For example, a health monitor may be configured to distinguish between non-recognizable activities that are similar to recognizable activity types (for which the health monitor is programmed to recognize) and non-recognizable activities that are not similar to recognizable activity types. In some embodiments, non-recognizable activities that are similar to recognizable activity types may be given a weighting value and evaluated according to metrics for the most similar recognizable activity. Non-recognizable activities that are determined to be not similar to recognizable activity types may or may not be given credit towards health benefits. Methods of health-benefit analysis are explained in further detail below for various categories of activities.

As a practical example of assessing similarity of activities, some exercise regimens that are set to music include a variety of repetitive body motions in the form of dance moves where new movements are created on a regular basis. Although an health monitor may be programmed to recognize some movement sequences, it may fail to recognize newly introduced movements. In some instances, motion and/or physiological data generated by a new movement may produce characteristic features $f_n$ that are similar to another activity recognized by the health monitor. The other activity may be a recognized dance movement, a recognized exercise (*e.g.,* jumping rope), or a movement executed on a specific exercise machine.

A determination of similarity or non-similarity of a detected activity as compared with a recognizable activity type may be based upon a value of the cost function $C_i$ or a value of a confidence in the match $PC_i$. For example, a threshold value may be selected for either of these quantities, and match evaluations yielding results greater than the threshold value and less than a matching value may be determined to be similar activities. Match evaluations yielding results less than the threshold value may be determined to be non-similar activities. As a further example that is not intended to limit the threshold and matching values, a threshold value of about 50% may be selected for determining similarity, and a matching value of about 80% may be selected for determining a positive match. Values of $PC_i$ that are 80 or greater would be considered a positive match, according to this embodiment. Values of $PC_i$ that are at least 50 but less than 80 would be considered similar, and values falling below 50 would be considered non-similar to recognizable activity types.

As noted above, in some implementations, feature sets for non-recognizable activities may be stored by a health monitor for subsequent identification by a user. For example, after downloading data from the health monitor to a computing device, the user may be prompted to identify an activity type associated with a feature set for which the health monitor was unable to recognize the activity. The identification may be based upon the time at which the activity was performed. In some embodiments, once identified by a user, stored data for the activity may be analyzed for health benefits. The health monitor may be subsequently updated to recognize the newly-identified activity type. In some implementations, there may be insufficient stored data for analysis of health benefits, and the health monitor is updated to recognize the activity during future use.

In some instances, *e.g.,* in an aerobics class or training camp where there may be various new motions, it may be inconvenient for a user of the health monitor to recall and identify each new movement. In such instances, a user may prefer to have the health monitor automatically associate activities with similar activities as described above, rather than store feature sets and prompt the user to identify each activity. According to some embodiments, a health monitor may be configurable by the user to either store feature sets for new non-recognizable activities for subsequent identification by the user and analysis, or automatically associate non-recognizable activities with similar activities as described above. A software setting may be provided to the user for configuring the health monitor's handling of non-recognizable activities.

In some embodiments, a health monitor may be configured to identify specific non-human activities, *e.g.,* precise repetitive circular motion representative of strapping a health monitor to a rotating object, repetitive back-and-forth motion representative of strapping a health monitor to a reciprocating object, *etc.* Non-human or non-animate activities may correspond to activities that exhibit features that could not be performed by a human. For example, the accelerometer data exhibits too high a degree of precision of repetitive motion over extended periods of time or excessive speeds, accelerations, or motions not sustainable by a human. In some implementations, a health monitor may detect such features, identify or classify the activity as non-human, and then terminate further processing of the activity data. Activities falling within the third classification (non-human activities) may be excluded from further analyses to determine a level of health benefit for the subject.

V. Health Benefit Analyses

In various embodiments, a health monitor may be configured to determine one or more levels of health benefit for at least some activities performed by the subject and detected by the health monitor. The inventors have recognized that some activity monitors can be adapted to produce "health credit data" that conforms to recognizable health standards, and that such adaptation can reduce inaccuracies and wide variability associated with step counting across different activity monitors. Health credits may, in some embodiments, be determined based upon activity type, and at least upon activity duration, intensity level of the activity, and standards established by a health entity. According to some embodiments, health credits may be determined from caloric burn rates, and caloric burn rates may be estimated more accurately using a combination of cardiac data and motion data rather than being based on cardiac data alone or motion data alone. Determination of health credits may provide a more reliable figure of merit for assessing health benefits derived from a subject's exercise program than conventional metrics such as number of steps taken, number of miles ran, number of minutes of exercise.

In some embodiments, health credits computed from exercise may be formulated as a point system, which can be used across all types of activities. For example, a subject may be given some portion or multiple of a health credit point, also referred to herein as a "health credit," for each minimum creditable time unit (MCTU) during which an activity is performed continuously at a particular intensity level. The number of points may depend upon the intensity level, and the intensity level and MCTU may be established by a recognized health organization. As one example, a subject may be given one health credit for each MCTU during which an activity is performed continuously at or above a first intensity level, and two points for each MCTU during which the activity is performed continuously at or above a second intensity level for the MCTU. In some implementations, a subject may be given a fractional point for each MCTU during which an activity is performed at a level below the first intensity level, *e.g.,* as an incentive to perform at least some activity.

In some implementations, computed health credits may be converted to total step counts using a standardized conversion formula, *e.g.,* to provide a comparison with legacy step-counting devices. Converting health credits, which may be computed more accurately than conventional step counts, to step counts may provide a more reliable comparison of step-count totals between different health monitoring devices.

The levels of demarcation for intensity and the MCTU may correspond with guidelines established by a recognized health organization. For example, the MCTU may be a one-minute interval, and the first intensity level may be a caloric burn rate that falls within a range between about 3.5 kilocalories (kcals/min) and about 7 kcals/min (a range identified as "moderate" activity by the Center for Disease Control (CDC) and World Health Organization (WHO)). The second intensity level may be a caloric burn rate that is at or exceeds about 7 kcal/min (a range identified as "vigorous" activity by the CDC and WHO). Other ranges and values may be used in other embodiments.

Health credits may be accumulated during a day or week, stored, and subsequently provided to a subject or physician as a record of the subject's exercise achievements for the time period. For example, a subject may perform activity in a day and be awarded 40 health credits for 40 minutes of moderate activity effort and 10 health credits for five minutes of vigorous activity effort, according to some embodiments. In some implementations, the same number of health credits may be awarded for same time intervals of moderate and vigorous activity, though the credits may be separated into "moderate" and "vigorous" bins. A record of health credits may provide a more convenient and accurate indicator of a subject's exercise and fitness level than a record of steps taken as calculated by different health monitors using different algorithms, for example.

In some embodiments, a health monitor may be configured to determine and record enhanced health credits (EHCs) for a subject. For example, the CDC and WHO have found that additional health benefits are obtained when an activity is performed continuously at a moderate or vigorous activity level for time intervals greater than about 10 minutes. In some implementations, a health monitor may determine when a subject performs one or more activities at moderate or vigorous activity levels for periods exceeding about 10 minutes and award EHCs to the subject. Further aspects and features will be described in association with the following methods and systems for determining health credits.

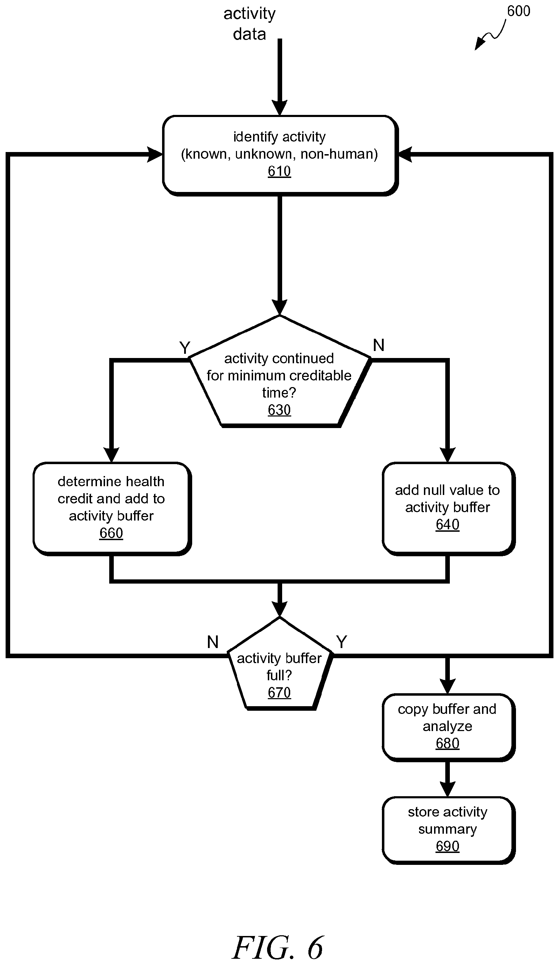

Referring now to FIG. 6, an illustrative example of a method 600 for determining health credits, according to some embodiments, is depicted as acts arranged in a flow chart. Embodiments of the method 600 may, for example, be implemented on a wearable health monitor, and may be used to determine health credits for a subject from one or more activities performed by the subject and detected by the health monitor. The flow chart provides an overview of some acts of just one example of a method 600, according to some implementations, and there may be more or fewer acts than illustrated in the figure in various embodiments.

According to some embodiments, activity data may be received from at least an accelerometer of a health monitor, and the health monitor may be configured to identify 610 an activity type from the data. In some embodiments, the activity data may comprise only motion data. In some implementations, activity data may further include physiological data received from one or more physiological sensors (*e.g.,* a cardiac waveform sensor). Once the system has identified 610 the activity, the system may determine 630 whether the identified activity has been continued for a minimum creditable time unit. If it is determined that the activity has been continued for a minimal creditable time, the system may determine 660 a health credit value for the activity and add a corresponding health credit value to an activity buffer. If the system determines 630 that an activity has not been continued for a minimum creditable time unit, the system may add 640 a null value to the activity buffer.

In various embodiments, the system may determine 670 whether the activity buffer is full. If it is determined that the activity buffer is not full, the system may return to a state of receiving activity data and identifying 610 an activity associated with the received activity data. If the system determines 670 that the activity buffer is full, the system may copy 680 the data from the activity buffer to another location and analyze the data. According to some embodiments the system may store 690 a summary of the activity that occurred during the filling of the activity buffer.

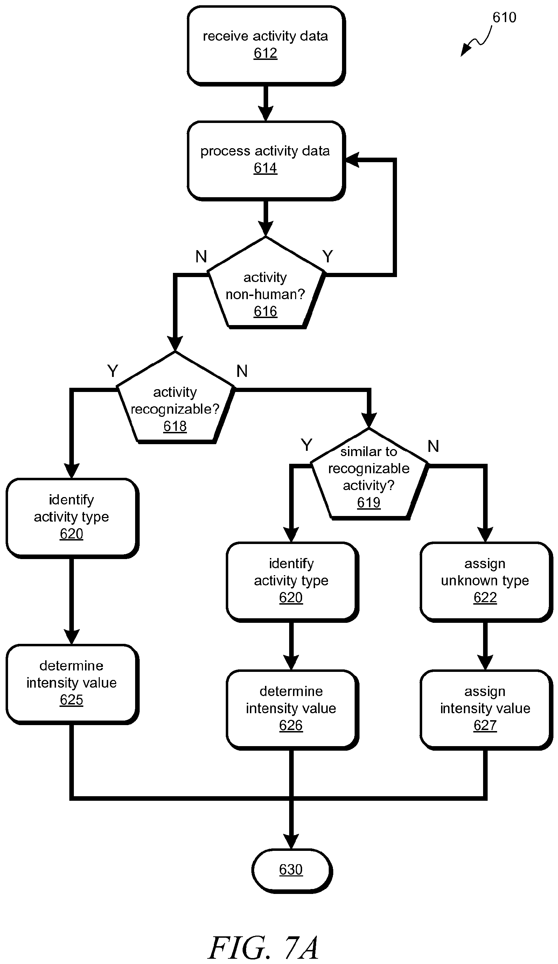

Figure 7A depicts a sub-process for identifying an activity 610, according to some embodiments. The sub-process for identifying an activity 610 may comprise receiving 612 activity data. For example, the received activity data may comprise motion data received from an accelerometer of the health monitor. In some embodiments, the received data may further comprise physiological data that may be received from one or more physiological sensors attached to a subject. The received activity data may include data that has been processed by a preprocessor 305 and feature generator 310, and may also include raw data, according to some embodiments. In some embodiments, the sub-process for identifying an activity 610 may further include processing 614 the activity data. The processing 614 of the activity data may be carried out as described above in connection with FIG. 3.

During processing of the activity data, an activity type may be identified, for example, as described above in connection with FIGS. 5A-5C. According to some embodiments, a detected activity may fall broadly into one of three classifications. For example an activity may be a recognizable activity that results in data produced by an accelerometer and/or physiological sensor having at least one associated feature set for which the health monitor is programmed to recognize. Examples of recognizable activities may be running, walking, biking, or swimming, though recognizable activities may not be limited to only these activities. Other recognizable activities may include different types of team sport activities. In some embodiments, the health monitor may detect an activity that is non-recognizable but has characteristics indicating that the activity was likely performed by a human subject. In some implementations, a health monitor may detect an activity that has nonhuman characteristics, for example, precise repetitive motion or excessive forces that are not likely sustainable by a human subject.

In some embodiments, a health monitor may determine 616 whether a detected activity is nonhuman. If it is determined that the detective activity is nonhuman, the health monitor may return to a state of receiving 612 activity data and processing 614 the activity data. According to some implementations, nonhuman activities may be ignored for further data processing. In some implementations, certain types of nonhuman activities may be identified by the health monitor (*e.g.,* motion in a washing machine, circular or reciprocating motion indicative of a machine). In either case, detected nonhuman activities would be recognized by the health monitor, so that no health credits would be awarded to a subject.

If it is determined that the detected activity may be associated with a human activity, the health monitor may attempt recognition 618 of the activity. In some embodiments, determining whether the activity is recognizable comprises an attempt by an inference engine 320 to determine an activity type. If the activity is recognizable, the system may identify 620 an activity type associated with the received activity data. In some embodiments, the system may further determine 625 an intensity value associated with the identified activity type. The system may then proceed to the step 630 of determining whether the activity is continued for a minimum creditable amount of time.

According to some embodiments, if a detected activity is not recognizable but exhibits characteristics indicating that it may be performed by a human subject, the system may determine 619 whether the activity is similar to a recognizable activity (*i.e.*, an activity for which the health monitor is programmed to recognize). If it is determined 619 that the activity is similar to a recognizable activity, the health monitor may identify 620 an activity type to associate with the detected activity, and determine 626 an intensity value for the detected activity. As just one example, received activity data may be determined to be most similar to a running activity, but does not include the requisite features for a definite identification of running.

Referring again to FIG. 7A, according to some embodiments, the similarity of an activity to a recognizable activity may be based at least in part on features and/or characteristics of motion data received from the accelerometer. In some cases, physiological data may be used in combination with motion data to determine 619 similarity of a non-recognizable activity to a recognizable activity. As an example, motion data, and in some cases physiological data, may be processed to generate feature sets as described above in connection with FIGs. 5A-5C. According to some embodiments, an activity that is determined to be similar to a recognizable activity may have a feature set for which at least some of the feature data falls within one or more membership functions for one or more recognizable activities. The data that falls within the one or more membership functions may not provide a high enough confidence level for making a definite match with any one recognizable activity. However, a non-recognizable activity may be determined as similar to a recognizable activity based upon the "nearness" of the measured features to a definite match for one of the recognizable activities. For example, an activity such as skipping may generate a feature set having values that are nearer to, or partially within, membership functions for running than to other membership functions, such as those for walking or biking. The activity of skipping may then be evaluated as being similar to a running activity.

An example of determining similarity is depicted graphically in FIGs. 5A through 5C. In this example, features 530-1, 530-2,... 530-n represent a set of features measured by the health monitor for an activity that is non-recognizable. The activity may be determined to be non-recognizable, because the measured features do not provide a percent confidence level that would give a definite match to either of the activities represented by the membership functions $M_1$ or $M_2$. In this example, feature 530-1 does not fall within a membership function $M_{1,1}$ or $M_{2,1}$. Measured feature 530-2 does fall within a membership function $M_{1,2}$ for the first activity, although the confidence level of featured 530-2 is low within this membership function. Measured feature 530-n does lie well within the membership function $M_{1,n}$, but the combined confidence of all the features for this activity may remain too low to provide a definite match to either activity $M_1$ or $M_2$. According to some embodiments, a health monitor may determine that the measured features 530-1, 530-2,... 530-n, as depicted in the example, are most similar to an activity represented by the membership functions $M_{1,1}$, $M_{1,2}$, and $M_{1,n}$. As can be seen from the graphs, these measured features are nearer to the membership functions for the first activity than they are to the membership functions for the second activity.

In other embodiments, a health monitor may calculate a distance between one or more measured features, for example, 540-1, and the nearest membership functions. The health monitor may determine which membership functions for an activity are closest to the measured features for the non-recognizable activity. If it is found that the measured features are nearer to membership functions for one activity than any other activity, then the health monitor may determine that the non-recognizable activity is similar to a recognizable activity for which the membership functions are nearest to the measured features for the non-recognizable activity.

According to some embodiments, an activity which is determined to be non-similar to any other activity may have a feature set for which the data does not fall within any membership function of any recognizable activity, or falls below a predetermined threshold value. An example of a non-similar activity is also depicted in FIGs. 5A through 5C. In this depiction features 540-1, 540-2,... 540-n represent measured features for an activity that may be determined 619 to be non-similar to any recognizable activity. In this case, the measured features all fall outside of membership functions for recognizable activities. For example, none of the measured features 540-1, 540-2,... 540-n fall within a membership function for a recognizable activity. According to some embodiments, a health monitor may determine such an activity to be non-recognizable and non-similar to a recognizable activity.

According to some embodiments, when a non-recognizable activity is found to be not similar to a recognizable activity, then a health monitor may assign 622 an unknown activity type for the activity. In some embodiments, a reserved character designation or bit sequence may be used for unknown activity types. For example, a bit sequence of all zeros (0000) may be assigned for an unknown activity type, though any other designation may be used. The reserved designation may later be used by the health monitor to identify periods of time in which unknown activities were performed, *e.g.,* for subsequently prompting the user to identify the activity for that period of time.

Referring again to FIG. 7A, after recognizing an activity or determining whether an activity is similar to a recognizable activity or is not similar to a recognizable activity, the health monitor may determine an intensity value for the activity. The determination 625, 626 of an intensity value may be done in any suitable manner, as described above for example. In some embodiments, motion data may be used to determine an intensity value for an activity. For example, motion data may be processed to determine a speed or rate at which an exercise is being performed, and an intensity value may be based upon or proportional to the determined speed or rate. In some embodiments, a motion waveform characteristic of an activity may be Fourier transformed to determine spectral power for the waveform. In some implementations, raw acceleration data may be squared and integrated, for example, to provide a value of spectral power. An intensity value for the activity may be based upon (e.g., proportional to) the measured spectral power. Additionally or alternatively, in some embodiments, determination of an intensity value may be based at least in part upon physiological data. For example, heart rate data (*e.g.,* R-R interval) and/or respiratory rate data (*e.g.,* encoded on R-waves over multiple beats) may be used to determine an intensity value for an activity. As just one example, based upon a repetition rate and/or physiological data (*e.g.,* heart rate, respiratory rate), a health monitor may associate or assign running at a pace having an equivalent heart rate and respiratory rate to the activity.

According to some embodiments, for non-recognizable activities, determination 626 of an intensity value for the non-recognizable activity may further comprise, for example, multiplying an intensity value determined for a similar activity based upon physiological data by a scaling factor. This may be implemented, for example, because the activity is not positively identified and is instead judged to be similar to a recognizable activity and is assigned an intensity value based upon the similarity and/or physiological data. The determination of an intensity value may be initially similar to or the same as a process used for the corresponding recognizable activity, but may then be scaled by a factor less than 1, according to some embodiments. In some embodiments, the scaling factor may be a constant value, or may be inversely proportional to a level of confidence in a match between the non-recognizable activity and associated recognizable activity. In some embodiments, the intensity value may be a reduced value compared to an intensity value for a recognizable similar activity. For example, the reduced value may comprise a scaling factor having a value of between about 50% and about 80% in some embodiments. In other embodiments, other values may be used for the scaling factor. Scaling the intensity value for a non-recognizable but similar activity can alter whether or not the activity is eligible to receive health credits.

If it is determined 619 that the received activity is not similar to a recognizable activity, the system may assign 622 an unknown activity type value to the detected activity. The system may further assign 627 an intensity value to the unknown activity type, in some embodiments. According to some implementations, the intensity value may be determined from one or a combination of: repetition rate, heart rate, and respiration rate. In some implementations, the assignment may additionally or alternatively be based on acceleration data, *e.g.,* repetition rate, detected speed, and/or power spectra of acceleration data. After concluding processing of data for an activity that is not recognizable, the system may proceed to the step of determining 630 whether the similar activity or unknown activity type is continued for a minimum creditable time unit.

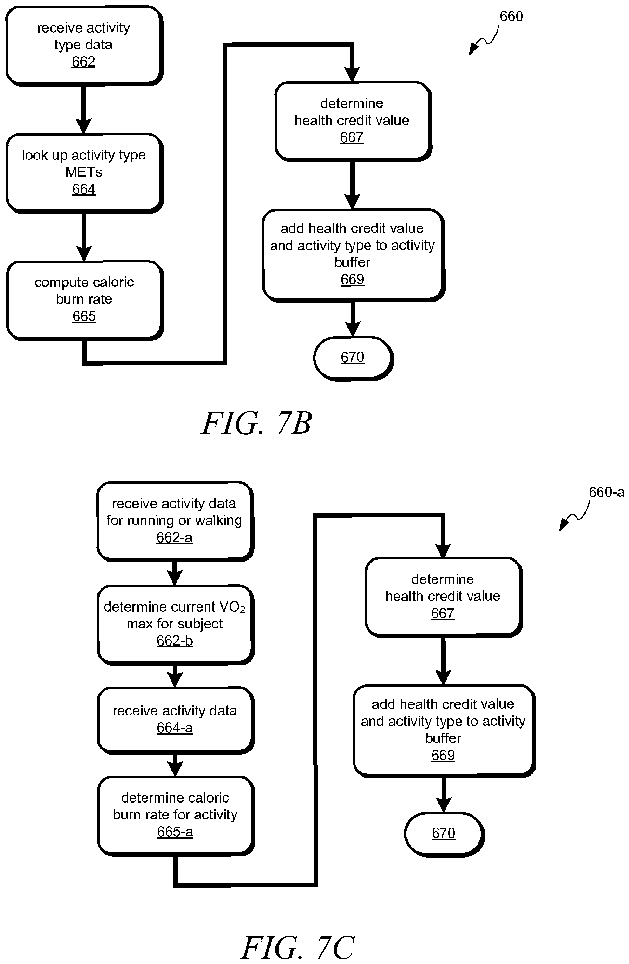

Referring now to FIG. 7B, an example embodiment of a sub-process of determining health credits 660 is depicted. The example method may be used to determine health credits using motion data only, *e.g.,* in embodiments where a health monitor may include motion detectors but lack physiological sensors. As depicted, the sub-process 660 may comprise an act of receiving 662 activity type data. The activity type data may, for example, comprise an identifier for an identified activity type and include an intensity level that was determined by the health monitor for the identified activity type. The sub-process 660 may further include looking up 664 metabolic equivalents (METs) for the activity type based at least in part on the determined intensity level at which the activity was performed. According to some embodiments, the METs may be determined from a lookup table as depicted in FIG. 8A.

The METs may provide a measure of calories burned per kilogram per unit time for the activity type and the activity intensity. In various embodiments, the METs may be obtained from a recognized compilation of metabolic equivalents for various activity types and intensities. An example compilation of METs can be found in "2011 Compendium of Physical Activities: A Second Update of Codes and MET Values," by Barbara E. Ainsworth et al., published by the American College of Sports Medicine, the entire contents of which are incorporated herein by reference.

According to some embodiments, a lookup data table 802 may comprise at least activity type data 810, intensity data 812, and metabolic equivalents data 814. For the example shown, the lookup table includes data for varying intensity levels of running and aerobics. The lookup data table may further comprise data for resting. In the example depicted, the data is represented as binary data, though in other embodiments any method to represent the data may be used.

According to some embodiments, once the activity type and intensity have been determined, they may be used to look up the METs in the metabolic equivalents data table 802. For example if the activity type is determined to be running, it may be represented by the binary sequence (0110). If the activity intensity is found to be representative of the subject running between 6 to 8 mph, the intensity level may, for example, be represented by a binary sequence (01000) representing 8 mph. The health monitor may then, using the activity type (0110) and intensity level (01000), use these values to search the metabolic equivalents data table 802 to find the associated metabolic equivalents (01011) for the activity. In this example, the number of METs for the identified activity would be 11 (METs). In some embodiments the METs may be rounded off to the nearest integer in the data table to reduce data storage space. In other embodiments fractional values may be stored in the data table, though this might require more memory on the health monitor.

Once the metabolic equivalents are determined, the health monitor may calculate 665 a caloric burn rate for the subject. The computation of caloric burn rate may, for example, include multiplying the METs by the weight of the subject. In some embodiments, the computation of caloric burn rate may further include converting units of time to minutes. Although FIG. 8A shows an illustrative example of a lookup data table 802 that may be used to determine METs and caloric burn for an activity according to some embodiments, other methods may be used to determine caloric burn and/or metabolic equivalents in other embodiments.

According to some embodiments, caloric burn may be computed using a combination of cardiac data and motion data. In some implementations, caloric burn rates may be computed for a subject that is walking or running using cardiac and motion data, and the obtained burn rates may be used as calibration values to estimate caloric burn rates for other activities.

For example, caloric burn may, in some embodiments, be computed more accurately based upon a subject's $VO_2$ max level and heart rate, rather than estimating caloric burn from heart rate data and a subject's weight, age, and exercise duration or from motion data and METs conversion. For example, when a subject's $VO_2$ max level is known, caloric burn may be computed with improved accuracy from the following expression.

$$Cal = 14.34[C_1 + C_2 HR + C_3 VO_2 + C_4 W + C_5 A]T \qquad (3)$$

where HR represents measured heart rate (in units of beats/min) of the subject during the performed activity, $VO_2$ represents a $VO_2$ max value (in units of mL kg$^{-1}$ min$^{-1}$) for the subject, W represents the subject's weight in kilograms, A represents the subject's age in years, and T represents the duration of the exercise in hours. The constants $C_n$ in EQ. 3 have been determined empirically for populations of subjects, and differ for male and female subjects. According to some embodiments, the constants $C_n$ may have values shown in Table 1. In some embodiments, other values may be used for the constants $C_n$ that are more specific to the subject, *e.g.,* determined from a population study for which the subject has greater commonality with the studied population.

Table 1

| constant | male | female |
|---|---|---|
| $C_1$ | -95.7735 | -59.3954 |
| $C_2$ | 0.634 | 0.45 |
| $C_3$ | 0.404 | 0.380 |
| $C_4$ | 0.394 | 0.103 |
| $C_5$ | 0.271 | 0.274 |

In some cases, a user of a health monitor may enter their $VO_2$ max value if they know its value. However, in most cases, a user does not know their $VO_2$ max value. In some cases, a value of $VO_2$ max for a subject may be determined from the following equation, for example, though other expressions may be used to compute $VO_2$ max in other embodiments.

$$VO_2 = \frac{m_1}{HR \times T_c} + b_1 \qquad (4)$$

EQ. 4 has been shown to provide an accurate estimate of $VO_2$ max for a population of subjects. (See, Weyand, P. G. et al., J. Appl. Physiol. 91: 451-458, 2001.) In this expression, $T_c$ represents foot contact time during running and the heart rate (HR) is determined during a period of time during which the foot contact time is approximately constant. A health monitor that includes an accelerometer located below the knee may be able to determine foot contact time in some embodiments. The quantities $m_1$ and $b_1$ may be gender specific and have values shown in Table 2. In some embodiments, other values of $m_1$ and $b_1$ may be used that are more specific to the subject, *e.g.,* determined from a population study for which the subject has greater commonality with the studied population.

Table 2

| | $m_1$ | $b_1$ |
|---|---|---|
| male | 34.4 | 11.1 |
| female | 30.9 | 10.3 |

In some implementations, foot contact time may be determined from speed of a subject, for example, when an accelerometer is located at a location other than below the knee. It has been found that foot contact time $T_c$ and speed $S$ are linearly related according to the following expression.

$$S = m_2 T_c - b_2 \qquad\qquad (5)$$

The values $m_2$ and $b_2$ depend upon whether the subject is running or walking and may have values shown in Table 3. In some embodiments, other values of $m_2$ and $b_2$ may be used that are more specific to the subject, *e.g.,* determined from a population study for which the subject has greater commonality with the studied population.

Table 3

|         | $m_2$ | $b_2$ |
|---------|-------|-------|
| walking | 3.15  | 945   |
| running | 2.58  | 258   |

In some embodiments, a value for $T_c$ may be determined according to EQ. 5, for example, from accelerometer data generated by a health monitor attached to a moving subject. The health monitor may be configured to identify a running activity of the subject as described above and the subject's speed. The health monitor may use the value of $T_c$ computed according to EQ. 5 in EQ. 4, for example, and a detected heart rate *HR* recorded during the activity interval of the subject for which $T_c$ was computed to compute a value of VO$_2$ max according to EQ. 4 for the subject. The computed value of VO$_2$ max may then be used in EQ. 3 to calculate a caloric burn for the subject. Basing caloric burn on calculated VO$_2$ max in this manner can improve the accuracy of computed health credits as compared to metrics based on only heart rate or accelerometer data.

According to some embodiments, a value of VO$_2$ max may be computed automatically by the health monitor for a subject at regular intervals (*e.g.,* daily, weekly, biweekly, *etc.*), so that the accuracy of computed caloric burn that is based upon VO$_2$ max (*e.g.,* computed according to EQ. 3) is updated regularly. Caloric burn may, for example, be computed using updated values of VO$_2$ max and EQ. 3 for various types of activities, whether or not the activities are recognizable, according to some embodiments.

Referring again to FIG. 7B, after caloric burn rates have been computed, the subprocess 660 may further comprise determining 667 health credit for the activity. In various embodiments, the health credit may be determined based upon at least the caloric burn rate that was maintained during a measured time interval for the activity. The health credit may be further based upon guidelines established by a health entity. For example, the Center for Disease Control and World Health Organization have determined that an exercise level yielding a caloric burn rate between about 3.5 kcal/minute and about 7 kcal/minute is found to be a first level of health-beneficial exercise. Accordingly, a health monitor may, for example, be configured to measure and process activity data in approximately one-minute time intervals, and give one health credit for each one minute of exercise at which the caloric burn rate falls within this range of caloric burn rates. The CDC and WHO have also found that exercise yielding caloric burn rates greater than about 7 kcal/minute provides a second level of health-beneficial exercise. Accordingly, a health monitor may, for example, be configured to give two health credits for each minute of exercise during which the caloric burn rate is equal to or greater than about 7.0 kcal/minute. In some embodiments, activities yielding a caloric burn rate below 3.5 kcal/minute may be awarded no health credits. In other embodiments, activities yielding a caloric burn rate below 3.5 kcal/minute may be awarded a partial health credit, for example, one-half credit. Other credit amounts and ranges of caloric burn rates may be used in other embodiments.

As may be appreciated from EQ. 3, in some embodiments it may not be necessary to identify an activity type to determine caloric burn and health credits. For example, an updated value of VO$_2$ max for a subject and a heart rate measured during an activity may be used to determine caloric burn rates for the subject for that activity. In some embodiments, a health monitor may, for a subject's convenience, identify or attempt to identify the activity performed. FIG. 7C is an illustrative example of an alternative method for determining health credits for a subject.

According to some embodiments, a method 660-a for determining health credits may comprise receiving 662-a activity data for running and analyzing the activity data to determine 662-b a current value of VO$_2$ max for a subject. The value of VO$_2$ max may be determined using values of heart rate and speed in EQS. 4 and 5, for example. A method 660-a may further comprise subsequently receiving 664-a activity data for a recognizable or non-recognizable activity performed by the subject. The activity data may include R-R intervals and/or cardiac waveform data from which a heart rate of the subject during performance of the activity can be determined. According to some embodiments, caloric burn rates may be computed 665-a for the activity, for example, using EQ. 3. In some implementations, a health monitor may also evaluate a power spectra of accelerometer data to verify that the heart rate is associated with physical activity performed by the subject. Health credits may be determined 667 from the calculated caloric burn rates and added 669 to an activity buffer, according to some embodiments.

Although $VO_2$ max may be computed from EQ. 4 based on running, in some embodiments, it may be computed from a different equation or set of equations based on another activity in other embodiments.

Equations other than EQ. 3 may be used, in some embodiments, to compute caloric burn and health credits. For example, the following equation may be used in some implementations.

$$Cal = 14.34[C_1 + C_2HR + C_3W + C_4A]T \qquad (6)$$

For this expression, the values of the constants $C_n$ may be as shown in Table 4. According to some embodiments, EQ. 6 may be calibrated for a subject using EQ. 3 to adjust some of the constants, *e.g.,* $C_2$ and $C_3$, so that the caloric burn is approximately the same for the two equations. In some embodiments, other values for the constants $C_n$ may be used that are more specific to the subject, *e.g.,* determined from a population study for which the subject has greater commonality with the studied population.

Table 4

| constant | male | female |
|---|---|---|
| $C_1$ | -55.0969 | -20.4022 |
| $C_2$ | 0.6309 | 0.4472 |
| $C_3$ | 0.1988 | -0.1263 |
| $C_4$ | 0.2017 | 0.074 |

As may be appreciated, a health monitor may award, or not award, health credits on a continual basis as exercise is being performed. For example, activity data may be streamed to the processor that analyzes the data on a continual basis to compute health credits. In various embodiments, the determination 660 of health credits may be made on a minute-by-minute basis, though any suitable time interval may be used.

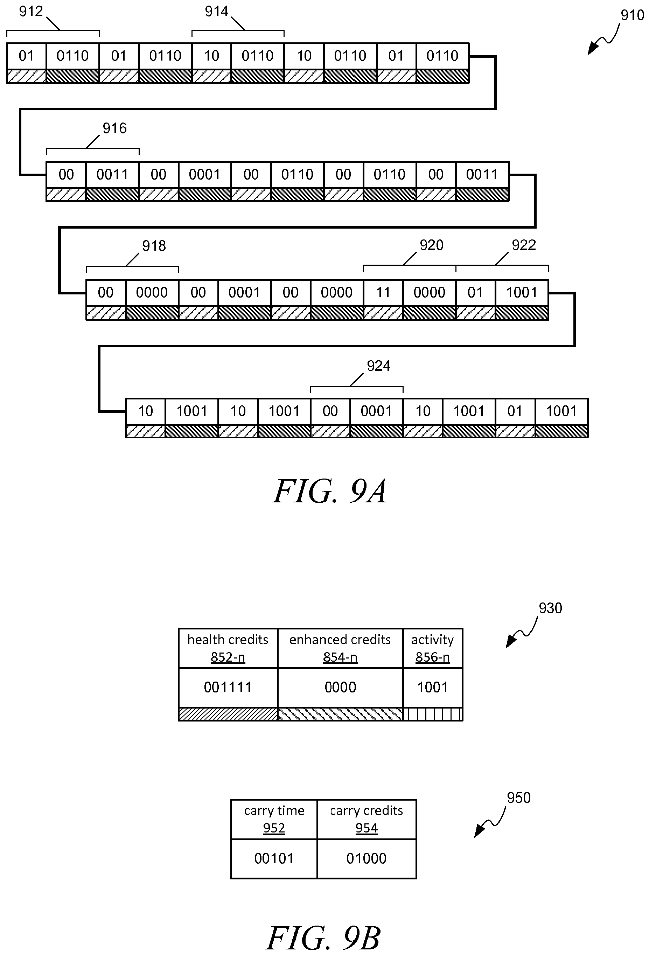

As the activity data is produced and analyzed and as the health credits are accumulated, the health monitor may generate a stream of health credit data 805, as depicted in FIG. 8B. The health monitor may temporarily store 669 the health credit data to an activity buffer. An example of an activity buffer 910 is depicted in FIG. 9A. The health monitor may, in some embodiments, further add 669 an indicator of activity type associated with any awarded health credit to the health-credit data stream 805.

The health-credit data stream 805 may, as just one example, comprise a series of bit sequences that are generated by the processor 110 and stored to temporary memory. The bit sequences may include at least two bit sequences having M bits and N bits for each data entry 820 in the stream 850. In some embodiments, each data entry 820 may contain information about the awarded health credit for an activity performed during the particular time interval, and may not include an activity identifier. In the example shown, two bits are used to represent the health credit value and four bits are used to represent the activity type. Accordingly there may be four values associated with health credit (*e.g.,* 0, 1, 2, 3), and eight values that may be used to identify different activity types for the embodiment shown. For the example shown in FIG. 8B, at least two activity types were identified during the generation of the health-credit data stream 805. The first activity type is represented by the bit sequence 0110, which corresponds to a running activity type according to the example shown in FIG. 8A. A second activity type, having the bit sequence 0001, corresponds to a resting state of the subject. During the filling of the buffer for two time intervals, the subject is awarded one health credit for the running activity, and during one time interval the subject is awarded two health credits for the running activity. No health credits are awarded during the resting interval. Other embodiments may use more or fewer bits to represent health credit and activity type and may include additional or less information in each data entry 820. In some embodiments, activity type may be identified only at the start or conclusion of an extended time interval over which the activity was performed.

After adding 669 health credits and activity type to the activity buffer, the health monitor made determine 670 whether the activity buffer is full. The activity buffer may be any suitable size and contain a plurality of data entries 820. The activity buffer may, for example, be sized to contain between 5 and 100 health-credit data entries 820. According to some embodiments, the activity buffer contains at least 10 health-credit data entries, and in some implementations may contain at least 20 data entries. FIG. 9A depicts an activity buffer 805 that contains 20 data entries where each data entry comprises a health credit value and an activity identifier, according to some embodiments. When it is determined 670 that an activity buffer is full, the activity buffer may be copied to additional storage, so that the copied contents of the activity buffer may be analyzed. In some embodiments, after the activity buffer is copied, the activity buffer may be erased or it may be overwritten with subsequent health-credit data.

In various embodiments, the size of the activity buffer 910 is small, so that it does not consume significant memory available at the health monitor, and so that the processor can readily process and compile the data between each refilling of the buffer. In some implementations, processing the data comprises reducing the data into a summary, so that the amount of memory required at the health monitor to store health-credit data and a record of activity is less than would otherwise be required to store data for every minute of operation of the health monitor.

Once the data from the activity buffer 910 has been analyzed, the health monitor may store 690 a summary of the activity buffer data. According to some embodiments, the summary of the activity buffer data reduces the amount of data represented in a filled activity buffer. The summary of the activity buffer data may form an on-board stored data stream 860 as depicted in FIG. 8C, according to some embodiments. The on-board stored data stream may comprise timing data 850, health-credit data 852, enhanced health-credit data 854, and activity type data 856, according to some embodiments. The on-board stored data stream 860 may comprise more, fewer, or different types of informational data in other embodiments. In some implementations, health-credit data 852, enhanced credits 854, and activity type data 856 may correspond to the analysis of at least one filled activity buffer 910. As the activity buffer 910 is repeatedly filled and analyzed, additional health credit, enhanced credit, and activity type data may be added to the on-board stored data stream 860.

In some implementations, timing data 850 may be added only to the beginning and/or end of the on-board stored data stream 860. In some cases, timing data 850 may be added at the beginning or end of each activity data summary corresponding to the analysis of one filled activity buffer 910. The timing data 850 may be used, for example, to keep track of when, and for how long, an activity was performed. In some embodiments, where the activity buffer 910 spans a fixed duration of time, each activity summary entry in the on-board stored data stream 860 represents a known duration of time. In such embodiments, timing data 850 may not be needed between each activity summary entry. Instead, the duration of an activity may be determined by the number of sequential entries in the on-board stored data stream 860. In some implementations, timing data 850 may be entered into the on-board stored data stream after breaks in activity, for example, when the health monitor is idle for a period of time or shutoff for a period of time.

Referring again to FIG. 9A, one embodiment of a filled activity buffer 910 is depicted. The example activity buffer shown spans 20 time intervals, and a data entry for each time interval includes two pieces of information (health credit values and activity type). For the example shown, the first data entry 912 indicates that a first activity type that is identified by the bit sequence (0110) (*e.g.*, running according to the example of FIG. 8A) received a health credit value of 1 (represented by the bit sequence (01)). The filled activity buffer 910 shows that the first type of activity was maintained for five time intervals. In the third time interval, represented as the third entry 914, the intensity level at which the subject performed the activity was increased, so that the subject received two health credits for the activity during that time interval. Two health credits were also awarded during the fourth time interval. In the sixth time interval, a second type of activity, defined by the bit sequence (0011) was performed by the subject. This activity (*e.g.,* walking) received no health credit. For example, the walking may have been at too slow a pace or may not have lasted for the full time interval. In the following time interval, the subject was in a resting state, represented by the bit sequence (0001). In the following three time intervals, the subject transitioned between a running state at a slow speed, which received no health credit values, and the walking state, which also received no health credits. During the eleventh time interval, represented by the eleventh data entry 918, the subject performed an activity which was not recognized by the health monitor. In this example, a non-recognized activity may be given a bit sequence of all zeroes. During this time interval, the subject may have exhibited some activity, such as stretching, but the health monitor could not determine or identify the type of activity, and/or the subject's heart rate may have been insufficient to award health credits.

During the fourteenth time interval, represented by the data entry 920, the subject exhibited appreciable activity that was not recognized by the health monitor. For example, the motion data and/or physiological data may have indicated that there was significant motion by the subject and an increased heart rate indicative of a high caloric burn rate. However, the health monitor could not determine identified the activity type. For example, during this period the subject may have been performing calisthenics or some type of warming-up exercise. Because of the detected motion and physiological data, the health monitor may be configured to award the subject a partial health credit or more. In some embodiments, a partial health credit may be indicated by the bit sequence (11), or another special bit sequence. In some cases, a partial health credit may be one half of a health credit. In the fifteenth time interval, the subject performed a different activity type identified by the bit sequence (1001), *e.g.,* biking. The subject then biked at a moderate activity level, and subsequently increased to a vigorous activity level for the following two time intervals. At the eighteenth time interval, represented by the data entry 924, the subject stopped temporarily receiving no health credits. During this time interval, for example, the subject may have been stopped by a traffic light. The subject then resumed biking for the remaining two time intervals as recorded in the activity buffer 910.

According to some embodiments, a filled activity buffer 910 may be analyzed to determine a total number of health credits received from the filling of the activity buffer, a number of enhanced health credits (EHCs) received, and/or to identify the predominant activity that occurred during the filling of the buffer. In some cases more than one activity type may be recorded with the analysis of the filled activity buffer 910. An analysis of a filled activity buffer 910 may result in an activity summary data entry 930, as depicted in FIG. 9B, that may be stored in longer term memory on the health monitor.

The activity summary data entry 930 may, for example, be one entry that is added to the on-board stored data stream 860 depicted in FIG. 8C. As described above, the data entry may include health credit data 852-n, enhanced health credit data 854-n, and activity type data 856-n. The health credits may be determined by summing the total number of health credits received during the filling of the activity buffer 910. In the example shown, the total number of health credits received during the 20 time intervals amounts to 15 health credits, which is represented by the bit sequence (001111). In the example shown, no enhanced credits were awarded.

In some embodiments, the health monitor may be configured to determine a predominant activity that was performed during the filling of the activity buffer. In some cases, the predominant activity may be an activity that was performed for the greatest number of time intervals within the activity buffer 910. In some implementations, the predominant activity may be determined as the activity receiving the most health credits during the filling of the activity buffer 910. In the example shown, the predominant activity is selected to be the activity for which the greatest number of health credits were received, which is biking in this example.

By compiling the filled activity buffer 910 into an activity summary data entry 930, the amount of data stored on board the health monitor may be appreciably reduced. In the example shown, one-hundred and twenty bits that are required for the filled activity buffer 910 may be reduced to fourteen bits for the activity summary data entry 930, representing about a 10:1 data reduction. Accordingly, activity data can be monitored and analyzed at a high resolution, *e.g.,* every minute, and meaningful information about the activity can be stored with significant data reduction. Additional data compression algorithms may additionally or alternatively be used to further compress the activity summary data entry in some embodiments. Data compression may be desirable so that longer intervals of time can be analyzed and recorded between downloadings of data from a health monitor that may require some manual interaction from the user.

In some implementations, analysis of a filled activity buffer 910 may further comprise tabulating carry time data 952 and carry credit data 954 as depicted in FIG. 9B. The carryover data 950 may be used, for example, for purposes of determining enhanced health credits 854-n, as will be explained in further detail below. As described above, enhanced health credits 854-n may be awarded when an activity is performed at or above a health-creditable intensity level continuously for a duration of time that spans multiple time intervals recorded in the activity buffer 910. For example, enhanced health credits may be awarded when an activity receiving health credits is performed continuously for at least 10 minutes in some embodiments, or at least 20 minutes according to some embodiments. In other embodiments, a time interval for receiving EHCs may be longer than or shorter than 20 minutes.

The determination of enhanced health credits 850-n may be carried out when the filled activity buffer 910 is analyzed. According to some embodiments, the health monitor may review each successive data entry in a filled activity buffer 910 to determine a number of successive data entries and time intervals that have received at least a partial or more health credits. If the number of successive data entries receiving a partial or more health credits is greater than a threshold number, then enhanced health credit may be awarded to the subject. As an example, each data entry in a filled activity buffer 910 may be representative of an activity performed for a time duration of one minute. If the filled activity buffer shows 20 data entries each receiving at least a partial health credit or at least one credit, then an enhanced health credit may be awarded to the subject. For the example and as shown in FIG. 9A, some data entries received no current health credits, and therefore an enhanced health credit is not awarded in this example.

Enhanced health credits can provide an additional piece of information about a subject's exercise regimen. For example, enhanced health credits can be used to readily determine whether the subject's exercise is piecemeal or extensive. Enhanced health credits can also provide a qualitative evaluation of a subject's overall fitness. For example, a subject that can sustain a health-creditable activity for an extended period of time would have a higher level of fitness than a subject who cannot and receives no enhanced health credits.

The awarding of enhanced health credits can be executed in any suitable manner. In some embodiments, enhanced health credits are awarded for each extended time interval that an activity is performed. For example, one EHC may be awarded for each twenty-minute extended interval that health-creditable exercise is continuously performed. In other embodiments, enhance health credits 854-n may be awarded for each health credit received during the extended period of exercise. For example, if during a twenty minute extended period of exercise a subject receives twenty four health credits, then the subject may receive 24 enhanced health credits. In some embodiments, separate point systems may be used for health credits and enhanced health credits. In yet other embodiments, the enhanced health credits 854-n may be representative of a percentage of the total health credits 852-n that received enhanced health credit. For example, during a twenty minute exercise interval a subject receives twenty eight health credits of which twenty one are enhanced health credits, then the enhanced credit value 854-n may represent a value of approximately 75%. In some embodiments, a percentage value may be rounded to the nearest 10's value.

As may be appreciated, extended intervals of activity may span more than one filled activity buffer 910. In the example shown in FIG. 9A the first activity, running, ended during the filling of the activity buffer, and a second activity, biking, began during the second half of filling of the activity buffer 910. For such cases, carry forward data 950 may be stored and used by a health monitor to carry forward, to the next filling of the activity buffer 910, information about an activity that was performed for an extended period of time during a previous filling of the activity buffer that could count toward enhanced health credits.

For the example of FIG. 9A, an extended period of time (from the sixth data entry 916 to the thirteenth data entry) passed during which no health credits were received. Accordingly analysis of the filled activity buffer 910 would yield no enhanced health credits. However, at the fifteenth time interval the subject began the activity identified as (1001), biking, which persisted until the end of the filled activity buffer 910, except for a brief interruption represented by the data entry 924. According to some embodiments, the health monitor may be configured to ignore the brief interruption, so that the biking activities may be carried forward as a continuous sequence of time intervals during which health-creditable activity was continuously performed. For this example, the carry time data 952 would reflect five time intervals that would count toward an extended time interval for the next filling and analysis of the activity buffer. For example, the five time intervals could be added to an extended time interval, commencing from the beginning of the next filling of the activity buffer, that could be eligible for receiving enhanced health credits. Carry credit data 954 may also be recorded to reflect the amount of health credits received during the carry time. In this example, eight health credits were awarded during the carry time.

The carry forward data 950 may be stored in a buffer temporarily, or may be stored in longer-term memory according to some embodiments. In some implementations, the carry forward data 950 may be erased or overwritten for each filling of the activity data buffer. The carry forward information 950 may comprise fewer or more bits than shown in FIG. 9B, according to some embodiments.

According to some embodiments, the health monitor may be tolerant of brief interruptions in exercise when determining whether or not enhanced health credit should be awarded to a subject. As described above in connection with FIG. 9A, the data entry 924, representing an interruption in biking activity (*e.g.,* being stopped by a traffic light), may be ignored by the health monitor when determining that the sequence of time intervals extending from the fifteenth to the twentieth time intervals were effectively continuously performed as health-creditable activity. In some embodiments, a health monitor may review the data entries in a filled activity buffer to determine the number of sequential data entries receiving no health credits. If the number of successive data entries receiving no health credits are fewer than a threshold number, then these data entries may be ignored when determining whether the activity surrounding these data entries were performed effectively continuously as health-creditable activity. According to various embodiments, the threshold number may be any value between zero and six, though higher numbers may be used depending on the time span of each data entry 912. As just one example, and referring again to FIG. 9A, the threshold number may be three. Accordingly, the data entries for the six through thirteenth time intervals represent a definite cessation of health-creditable activity. Any carry time occurring before this cessation would be nullified or not added to an extended interval occurring after the cessation. However, the data entry 924 represents an interruption (less than three intervals) that can be ignored by the health monitor for purposes of determining enhanced health credit. Any carry time and/or or extended time occurring before the interruption can be added to an extended activity interval occurring after the interruption for purposes of determining enhanced health credits.

In some embodiments, a time interval receiving a partial health credit may be treated as a time interval that receives no health credit for purposes of determining enhanced health credit. For example, a time interval receiving a partial health credit may count towards a cessation or interruption in activity. In other embodiments, a time interval receiving a partial health credit may be treated as a time interval receiving one or more health credits for purposes of determining enhanced health credit. For example, a time interval receiving a partial health credit may count towards an extended time interval during which health-creditable activity is performed.

It will be appreciated that health credits may be converted to step counts, in some embodiments, for comparison with legacy systems. For example, health credits that are determined from caloric burn rates can be expressed in terms of METs. From the METs value, a walking speed may be determined for a subject, and from the walking speed and subject's gate (as may be determined with the motion sensor), equivalent steps taken during an exercise interval to obtain the same METs result may be determined. In some embodiments, a health monitor may be configured to accumulate equivalent steps for all activities performed by the subject that receive health credits.

## VI. Additional Aspects of Processing Activity Data

Determining parameters, such as distance and speed of a walking or running person, that accurately represent a detected activity may be difficult without proper calibration techniques. Thus, in some embodiments, to ensure that a health monitor 100 provides data that accurately reflects various parameters associated with a detected activity, activity-dependent calibration factors may be employed, *e.g.,* such factors may be used by an activity engine 340-m when computing activity-related data from data received from motion detection and preprocessing circuitry. Activity-dependent calibration factors for each activity may, for example, be maintained and updated in memory 120.

In some implementations, calibration factors may be used in one or more equations used by an activity engine 340-m to compute a measure of intensity of the activity. An example of such an implementation is disclosed, for example, in U.S. patent No. 4,578,769 (incorporated by reference above) which describes deducing a runner's speed based upon foot contact time that is detected by a sensor placed in footwear. In some embodiments, there may be a number of different calibration factors needed for a health monitor configured to recognize a number of different activity types. Such calibration factors may, for example, be determined ahead of time, *e.g.,* through laboratory testing and experimentation, and then loaded into memory 120 of a health monitor 100 prior to its use.

In some embodiments, calibration may be performed in conjunction with a user's review of the data and user input. For example, a user may jog for 2.0 miles and record a time, 14 minutes, 0 seconds (14:00), that it took to jog the two miles. A health monitor may, for example, use a pre-defined calibration technique to identify the activity as running and the activity engine 340-m may compute a running pace of 6:50 minutes/mile. In such an implementation, the user may then, via a computer-based interface with the health monitor, execute a calibration routine wherein the user may first select the identified activity and computed pace, and then enter a known pace for the activity. The system may then adjust or replace an internal calibration factor used by the health monitor 100 with a new calibration value for that activity. In this manner, calibrations for various activities can be made specific to individual users of the health monitor, which may improve the accuracy of the device for each user.

In some embodiments, a health monitor 100 may be additionally or alternatively configured for automatic calibration or self-calibration for one or more activities. Such calibration routines may be executed for one or more recognizable activities. As just one example, self-calibration for running will be described. When a health monitor includes an accelerometer that is placed on the foot or ankle, the accelerometer will temporarily come to rest along the direction of running (taken as *x*-directed in this example) as the foot plants on the ground. When the foot is planted, the *x*-directed velocity of the accelerometer is zero, and this can serve as a reference point for calibration. When the foot next plants, the *x*-directed velocity returns again to zero. By integrating the *x*-directed acceleration data twice, a distance between the two successive foot plants can be determined. The distance may be corrected using *y*- and *z*-directed acceleration values, since the orientation of the accelerometer changes as the foot moves forward. Once the distance is determined, a time between the foot plants may be determined from an internal clock of the processor 110, for example. The time and distance may then be used to calculate a velocity of the runner, or walker. The velocity may be determined from two successive foot plants, or more to obtain an averaged value, and the process of determining velocity may be repeated at separated intervals of time. In some embodiments, the calculated velocity may be used to update or correct internal calibration values used by a health monitor 100. For example, the calculated velocity may be used to correct a calibration value used for estimating running speed based on foot contact time.

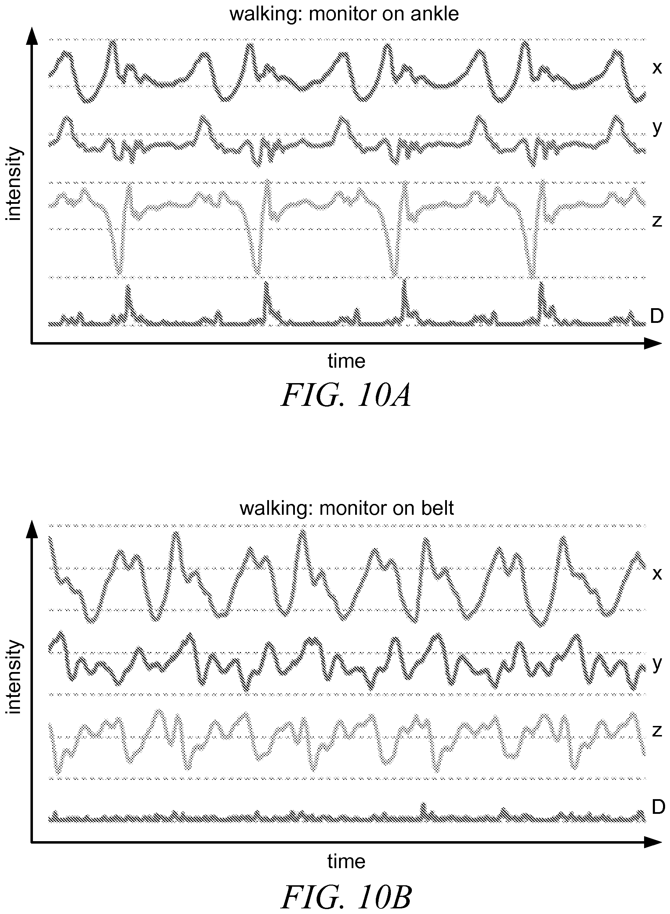

Calibrations may additionally or alternatively be used by the health monitor in a different manner, and such calibrations may be referred to as location-dependent calibrations. For example and with regard to running or walking without being limited to only these activities, when a health monitor 100 is placed on the ankle or foot, for example, a more precise measurement can be made of the activity than if the monitor were worn on the belt or placed in a trouser pocket. This can be seen, for example, in the raw accelerometer data traces of FIGS. 10A-10C. When the monitor is worn on the ankle (FIG. 10A), the *z* and *x* waveforms are more pronounced than when the monitor is worn on the belt (FIG. 10B). The timing of the foot strike and/or foot contact time can be determined more accurately using the data from a health monitor worn on the ankle or foot.

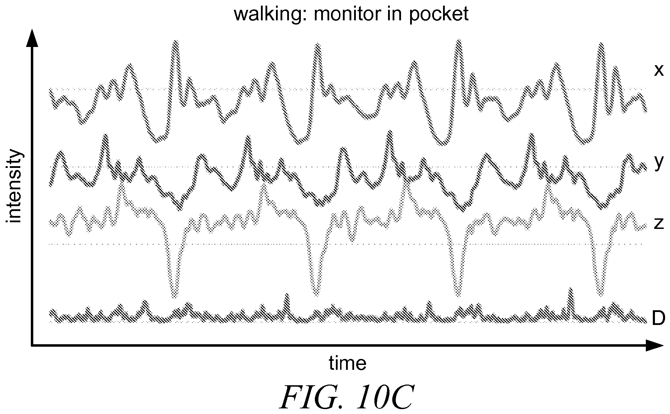

In various embodiments, a health monitor may additionally or alternatively be configured to recognize a type of activity independent of the location of where the motion sensor is worn, and is further configured to identify where the motion sensor is worn for the activity. Just as the data traces of FIG. 10A can be identified as walking by inference engine 320 as described above, the traces of FIGS. 10B may be identified as walking where the motion sensor is worn on a belt, and the traces of FIGS. 10C may be identified as walking where the motion sensor is located in a pocket. For example, the traces of FIG. 10B may generate characteristic features $f_n$ belonging most closely to one or more membership functions that would identify the activity as "walking, motion sensor on belt."

In some embodiments, when an activity is identified where a health monitor includes an accelerometer mounted in a non-optimal location, a different calibration or scaling value, or values, may be used by activity engine 340-m to compute one or more parameters associated with the activity, according to some embodiments. For example, different calibration values may be associated with each identifiable activity and motion sensor location. In other embodiments, when an activity is identified where the motion sensor is mounted in a non-optimal location, information that was gathered from prior use of the health monitor, when the motion sensor was mounted in a more optimal location, may additionally or alternatively be used to infer or estimate parameters of the activity with the motion sensor in the non-optimal location. For example, walking data gathered when the motion sensor is worn on an ankle may be used to determine stride lengths that correspond to different walking step frequencies or cadences. Then, when the motion sensor is worn at a non-optimal location (*e.g.*, a belt or pocket), a detected cyclic frequency or cadence may, for example, be used in conjunction with the previously-obtained data to infer or estimate a stride length for the activity. The estimated stride length may be user-specific. In some implementations, different calibration techniques and/or calibration values may be associated with each identifiable activity and motion sensor location.

In some embodiments, characteristics of an activity may additionally or alternatively be inferred by a health monitor from prior high quality data, and an intensity for the activity may be computed accordingly. Returning again to the example of FIGS. 10A-10C, the health monitor 100 may, for example, store in memory 120 or provide for storage in an external memory device one or more samples of high quality data (FIG. 10A) when such data is collected and the motion sensor is worn in an optimal or near-optimal location for characterizing the activity. In some embodiments, when the activity is repeated and the inference engine identifies the activity but with the motion sensor worn in a non-optimal location (FIG. 10B or 10C), the health monitor may recall from memory, higher quality data with a cadence that matches the currently sensed activity. The higher quality data may, for example, be repeatedly provided to activity engine 340-m for subsequent processing. As the currently sensed cadence changes, different samples may, for example, be retrieved from storage. In some embodiments, the samples retrieved from storage may depend on additional values of the currently sensed signals other than cadence, *e.g.,* peak values, widths of peaks, minimum values.

In some embodiments, a health monitor may additionally or alternatively provide a measure of confidence along with data output by an activity engine 340-m. For example, the monitor may indicate a confidence level in the recognition of the activity (*e.g.,* > 90% confidence, > 75% confidence, > 95% confidence), and may also indicate a level of quality of the data (*e.g.,* best, fair, poor). Confidence may be determined, for example, by how central each measured feature characteristic falls within a membership function, or based upon a calculated value of the cost factor (*e.g.,* value calculated in accordance with EQ. 2) for an activity, or how well a measured pattern matches a reference pattern (*e.g.,* using a least-means-squared difference algorithm). Quality of the data may be determined, for example, based upon an identified location of the health monitor's motion sensor when worn by the user during the identified activity.

As described above, in some embodiments, the calibration values, characteristic features, membership functions, and/or computation algorithms used by a health monitor 100 may be added and/or revised when the device is interfaced with a computer via transceiver 140. It should thus be appreciated that in such embodiments the health monitor may be personalized to become more accurate for a given activity, for a given location of sensors being worn, and/or for a particular user. For example, when the device detects activity data that cannot be recognized by the inference engine 320, the device can log the acceleration data, and optionally physiological data, along with any related characteristic features generated from the data, the time, and duration of the non-recognized activity. In some embodiments, when subsequently in communication with an external device having a user interface, such as a computer, smart phone, PDA, or similar device, the system may, for example, present a query to the user to identify the activity, intensity of effort, and/or location of the motion sensor. In such embodiments, the information may, for example, be returned to the device, and a new membership function, features, and/or identification algorithm may be defined for the activity. The membership function and/or identification algorithm may, in some embodiments, be produced external to the health monitor and downloaded. In some embodiments, one or more new activity engines 340-m may additionally or alternatively be added for the purpose of personalizing a health monitor 100.

In some embodiments, a health monitor 100 may be useful for broad community challenges, allowing for users to be able to readily compare themselves to each other. A health monitor may, for example, be used for more accurately handicapping users of different performance capabilities. Some embodiments of the health monitor 100 may, for example, allow healthcare providers, insurance companies and employers to more accurately assess fitness levels, exercise regimens, and health benefits from exercise for individuals, and provide appropriate incentives accordingly.

According to some embodiments, a health monitor may be configured to compute a fitness metric that is indicative of a subject's overall fitness and/or health level. For example, a health monitor may collect activity data, which may include physiological data (*e.g.,* blood oxygenation data, respiratory data, cardiac data) and health credit data, and compute a fitness metric from the results using a standardized algorithm. In some embodiments, the fitness metric may be representative of a $VO_2$ max value for the subject or may be a recognized or standardized fitness or health index (HI). According to some embodiments, a fitness metric $F_m$ may be computed by a health monitor using the following formula.

$$F_m = \beta \frac{P}{R \times HR} \qquad (7)$$

In EQ. 7, $\beta$ represents a proportionality constant, $P$ may represent a pace, or equivalent pace calculated from a different activity via metabolic equivalents, $R$ may represent a respiratory rate, and $HR$ may represent a heart rate. The fitness metric in EQ. 7 has been formulated such that a higher number indicates a better level of fitness. For example, for a given pace $P,$ an individual with a lower respiratory rate $R,$ and lower heart rate $HR$ will be a healthier or more fit individual, since he is able to achieve a same pace more efficiently (taxing the cardiovascular system by a lesser amount).

Other fitness metrics may be computed additionally or alternatively. As just one example, an aerobic fitness

metric $F_a$ may be computed from the following equation for a subject.

$$F_a = 1/(T_c \times HR) \qquad\qquad (8)$$

In this expression, $T_c$ represents foot contact time, which may be determined from running speed according to EQ. 5, and *HR* represents heart rate.

Some embodiments may use other fitness metrics, or a metric where a lower number, rather than a higher number, indicates a better level of fitness. Other factors (*e.g.,* body mass index, blood pressure, blood oxygenation, resting metabolic rate, caloric burn rate, metabolic rate) may be used in addition to, or alternative to, those shown in EQ. 7 to determine a fitness metric. In some embodiments, a fitness metric may further include an assessment of a subjects health credits and/or enhanced health credits accumulated over a period of time (*e.g.,* a week, month, several months, a year, *etc.*) A fitness metric may comprise a weighted combination of several fitness indicators. A fitness index such as that shown in EQ. 7 or EQ. 8 may provide a snapshot of a subject's fitness level and/or health.

Other fitness metrics may also be determined with a health monitor that is configured to provide cardiac and motion data. As just one example, a health monitor may process activity data to determine when a subject is sitting or in a prone resting state, and process cardiac data during a resting state to determine a subject's resting heart rate (RHR).

In some embodiments, motion data may be processed by the health monitor's processor to determine when a subject is in an active state and performing an activity at near maximum capacity. Performance of an activity near maximum capacity may be determined, for example, from heart rate, speed of the subject, and/or repetition rate corresponding to the activity. In some implementations, a level of activity near maximum capacity may be indicated by a detected heart rate that is greater than 75% of a maximum heart rate detected for the subject, or greater than 85% of a maximum heart rate detected for the subject in some embodiments, or greater than 90% of a maximum heart rate detected for the subject in other embodiments. A health monitor may then be configured to determine from motion data when the subject stops the activity and assumes a resting or recovery state. The health monitor may then process cardiac data following the cessation of the activity to determine a heart-rate recovery time for the subject. A shorter recovery time may indicate a higher level of fitness. According to some embodiments, determination of heart-rate recovery times may be executed automatically by the health monitor following any performance of an activity near maximum capacity by the subject.

According to some embodiments, heart rate variability (HRV) may be determined by a health monitor during periods of activities performed at different intensities to assess levels of stress induced on a subject by the activity. Less reduction in HRV for higher levels of effort in an activity may be indicative of a more fit subject. In some implementations, a health monitor may be configured to record and process cardiac waveforms for extended intervals of time (*e.g.,* greater than 30 second intervals) to determine low-frequency and high-frequency spectral properties of the waveforms. Low-frequency (LF) components may, for example, comprise frequencies between approximately 0 Hz and approximately 0.15 Hz. High-frequency (HF) components may, for example, comprise frequencies between approximately 0.15 Hz and approximately 0.4 Hz. According to some embodiments, a ratio of LF/HF spectral power may be computed during periods of activities performed at different intensities to assess levels of stress induced on a subject by the activity.

In some implementations, a health monitor may be configured to evaluate HRV and/or LF/HF spectral power to assess a condition of a subject. As just one example, a health monitor may evaluate LF/HF spectral power in the morning when an athlete has awaken, but is still in a prone position. A value of the LF/HF spectral power taken at this time may indicate that the athlete has, or has not, fully recovered from a prior day's workout. In some embodiments, a ratio between about 0 and 0.15 may indicate that an athlete has not fully recovered from the previous day's workout. The value may also be used to indicate, for example, a level of activity that can be performed by the athlete during the day at a lowered risk of injury to the athlete.

As another example, a value of an LF/HF spectral power ratio may indicate that an athlete has, or has not, warmed up sufficiently before a more taxing workout. For example, a LF/HF spectral power ratio calculated by the health monitor to be greater than 0.4 may indicate that an athlete has not warmed up sufficiently, and may further indicate that a product of HR*$T_c$ may not be accurate for assessing various fitness parameters. In some implementations, a value of LF/HF spectral power may be used to assess when subjects having a particular ailment (*e.g.,* CHF, COPD, advanced stages of cancer or diabetes, *etc.*) is in a state of stress. For example, a ratio between about 0 and 0.15 may indicate that a patient is in a state of stress.

One or more fitness metrics or a health index value may provide ways in which to monitor health conditions of subjects. For example, certain ailments may adversely affect a fitness index for an individual. Ailments that can adversely affect a fitness index include, but are not limited to, COPD, CHF, arthritis, dementia, diabetes, depression, PAD, hypertension, and obesity. In type two diabetes (T2D) there have been studies that indicate that an increase in a subject's $VO_2$ max level directly correlates to reduced dependency in medication. For COPD patients, a fitness index (*e.g.,* $VO_2$ max) may provide a diagnostic marker showing the stage and progress of the disease.

Some studies have shown that physical activity may help correct a disease state, reduce medication, or delay the onset or progress of a disease state. The CDC/HHS recommends moderate to vigorous activity about 150 minutes a week for the normal population. Although some activity monitors may monitor basic parameters of an activity, many conventional monitors cannot provide an objective viewpoint or metric as to how well a patient or athlete is progressing on their fitness journey. A health monitor configured to compute health credits and fitness metrics as described above may provide a convenient device for measuring and tracking a subject's fitness level on a daily basis.

There is currently little or no guidance for ailing individuals as to what level of exercise is most effective for their condition. Although workout tables have been generated for various ages, *e.g.,* listing maximum recommended heart rates by age and gender, these tables do not take into account disease states or medication regimens for individuals. Thus, with regard to exercise-based treatment of an ailments, one does not know whether the level of exercise is too little to be effective, or too much to pose an additional health risk. A health monitor that can be networked can provide data for large populations of users, from which guidelines for exercise may be established based on age, gender, ailment, and stage of ailment. Accumulated statistical data can be used to update health monitors with recommended exercise guidelines, in terms of health credits and enhanced health credits in some embodiments, for various health conditions of a subject.

When treating an ailment, there may be times where medication prescribed to treat a disease causes a detrimental effect on the subject, *e.g.,* lowering the body's ability to metabolize oxygen. Monitoring a fitness index that correlates to blood oxygenation for a subject afflicted with one or more of these ailments may provide a convenient way to assess effectiveness of various types of treatment, including exercise and pharmaceutical therapy, and to track recovery of the subject. For example, a health monitor that summarizes exercise in terms of health credits and/or a fitness index may allow a physician or individual to see, almost immediately, any impact a prescribed treatment may have on the human engine (heart-lung-circulatory system).

Although the above examples for determining health benefits are framed primarily in terms of two activity intensity levels (moderate and vigorous) for determining health credits and enhanced health credits, other embodiments may use additional or fewer activity intensity levels and criteria for determining health credits, enhanced credits, and fitness metrics. For example, a health entity may further refine intensity levels and criteria standards for evaluating health benefits from activities, so that the ranges and number of intensity levels may be altered according to a health entity. In various embodiments, a health monitor may be readily reprogrammed to accommodate such changes. Additionally, gradations of enhanced health credits may be implemented in some embodiments. For example, different values or point systems for enhanced health credits may be awarded based upon one or both of activity intensity and length of extended duration of the activity. As an example, an additional point system may be used for health-creditable activities performed continuously for 40 minutes, for example, so that a subject or physician can better assess an endurance fitness metric for the subject.

All literature and similar material cited in this application, including, but not limited to, patents, patent applications, articles, books, treatises, and web pages, regardless of the format of such literature and similar materials, are expressly incorporated by reference in their entirety. In the event that one or more of the incorporated literature and similar materials differs from or contradicts this application, including but not limited to defined terms, term usage, described techniques, or the like, this application controls.

The section headings used herein are for organizational purposes only and are not to be construed as limiting the subject matter described in any way.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, and/or method described herein. In addition, any combination of two or more such features, systems, articles, materials, and/or methods, if such features, systems, articles, materials, and/or methods are not mutually inconsistent, is included within the inventive scope of the present disclosure.

The above-described embodiments of the invention can be implemented in any of numerous ways. For example, some embodiments may be implemented using hardware, software or a combination thereof. When any aspect of an embodiment is implemented at least in part in software, the software code can be executed on any suitable processor or collection of processors, whether provided in a single computer or distributed among multiple computers.

In this respect, various aspects of the invention, *e.g.,* feature generator 310, preprocessor 305, inference engine 320, activity engines 340-m, and data service 360, and versatile sensor networking functionality, may be embodied at least in part as a computer readable storage medium (or multiple computer readable storage media) (*e.g.,* a computer memory, one or more floppy discs, compact discs, optical discs, magnetic tapes, flash memories, circuit configurations in Field Programmable Gate Arrays or other semiconductor devices, or other tangible computer storage medium or non-transitory medium) encoded with one or more programs that, when executed on one or more computers or other processors, perform methods that implement the various embodiments of the technology discussed above. The computer readable medium or media can be transportable, such that the program or programs stored thereon can be loaded onto one or more different computers or other processors to implement various aspects of the present technology as discussed above.

Various aspects of a health monitor described above may be implemented in hardware, software, firmware, or a combination thereof. For example, any of the operational aspects of a health monitor which involve processing data, handling data, and/or communications may be implemented as stored machine-readable instructions that are executable by a microprocessor and embodied on at least one tangible, computer-readable storage device. The instructions may be executed or placed in operation on a digital processor of a health monitor. In some implementations, instructions may be placed in operation on a central hub or server that operates in combination with operation of a health monitor.

The terms "program" or "software" are used herein in a generic sense to refer to any type of computer code or set of machine-executable instructions that can be employed to program a computer or other processor to implement various aspects of the present technology as discussed above. The term "processor" may be used to refer to at least one microprocessor, microcontroller, or any suitable programmable logic device including, but not limited to field programmable gate arrays. Additionally, it should be appreciated that according to one aspect of this embodiment, one or more computer programs that when executed perform methods of the present technology need not reside on a single computer, processor, or microcontroller, but may be distributed in a modular fashion amongst a number of different computers, processors, or microcontrollers to implement various aspects of the present technology.

Computer-executable instructions may be in many forms, such as program modules, executed by one or more computers or other devices. Generally, program modules include routines, programs, objects, components, data structures, *etc.* that perform particular tasks or implement particular abstract data types. Typically the functionality of the program modules may be combined or distributed as desired in various embodiments.

Also, the technology described herein may be embodied as a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, may be used to mean "at least one."

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); *etc.*

As used herein in the specification and in the claims, "or" should be understood to have the same meaning as "and/or" as defined above. For example, when separating items in a list, "or" or "and/or" shall be interpreted as being inclusive, *i.e.*, the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); *etc.*

The claims should not be read as limited to the described order or elements unless stated to that effect. It should be understood that various changes in form and detail may be made by one of ordinary skill in the art without departing from the spirit and scope of the appended claims. All embodiments that come within the spirit and scope of the following claims and equivalents thereto are claimed.

Further aspects of the invention are set out in the following numbered paragraphs, which form part of the description of this application and do not constitute claims thereof.

1. A health monitor configured to be supported by a subject, the health monitor comprising:

an accelerometer configured to generate motion data in response to a first activity performed by the subject;
a cardiac sensor configured to detect at least a heart rate of the subject during performance of the first activity;
an on-board power source configured to provide power to the accelerometer and the cardiac sensor; and
a processor configured to calculate a first caloric burn rate for the first activity performed by the subject according to a first method based upon data from both the accelerometer and the cardiac sensor.

2. The health monitor of paragraph 1, wherein the processor is further configured to determine at least one power conservation mode of the health monitor based upon data received from the cardiac sensor.

3. The health monitor of paragraph 2, wherein the at least one power conservation mode comprises a mode in which power is reduced to at least the cardiac sensor for an interval between T-wave and P-wave portions of successive cardiac cycles.

4. The health monitor of any of paragraph 1-3, wherein the processor is configured to calculate the first caloric burn rate using values of heart rate and speed of the subject or values of heart rate and foot contact time of the subject.

5. The health monitor of any of the preceding paragraphs, wherein the processor is configured to calculate the first caloric burn rate using values of heart rate and a $VO_2$ max value computed for the subject.

6. The health monitor of any of the preceding paragraphs, wherein the processor is further configured to calibrate a second method for determining caloric burn for a subject performing an activity, wherein the calibration is based upon results obtained from the first method.

7. The health monitor of paragraph 6, wherein the second method comprises determining caloric burn based upon heart rate and not based upon data from the accelerometer.

8. The health monitor of any of the preceding paragraphs, further comprising a timer, wherein the processor is further configured to:

calculate a second caloric burn rate for a second activity performed by the subject for a first time interval of a sequence of first time intervals; and
determine whether the second caloric burn rate falls within at least one recommended range of values proscribed by a health entity for the duration of the first time interval.

9. The health monitor of paragraph 8, wherein the health entity is the Center for Disease Control or the World Health Organization.

10. The health monitor of paragraph 8, wherein the health entity is a physician, a qualified medical personnel, or a professional trainer.

11. The health monitor of any of paragraphs 8-10, wherein the processor is further configured to verify performance of the second activity from a power spectrum of the motion data.

12. The health monitor of any of paragraphs 8-11, wherein the processor is further configured to determine the intensity level of the second activity from a heart rate and/or respiratory rate of the subject.

13. The health monitor of any of paragraphs 8-12, wherein the processor is further configured to:

record a first credit value for each first time interval that the caloric burn rate falls within a first recommended range of the at least one recommended range for the duration of the first time interval; and
record a second credit value for each first time interval that the caloric burn rate falls within a second recommended range of the at least one recommended range for the duration of the first time interval.

14. The health monitor of paragraph 13, wherein the first recommended range of the at least one recommended range is between approximately 3.5 kilocalories per minute and approximately 7 kilocalories per minute and the second recommended range of the at least one recommended range includes values greater than approximately 7 kilocalories per minute.

15. The health monitor of paragraph 13 or 14, wherein the processor is further configured to:

identify a type of the second activity performed by the subject from among a plurality of activity types for which the health monitor is configured to recognize; and
identify non-human activities.

16. The health monitor of any of the preceding paragraphs, wherein the processor is further configured to:

determine, from data received from the accelerometer, a period of increased activity performed by the subject; and
determine a heart-rate recovery time following cessation of the activity.

17. The health monitor of any of the preceding paragraphs, wherein the processor is further configured to calculate heart rate variability or a LF/HF spectral power ratio from data received from the cardiac sensor.

18. The health monitor of any of the preceding paragraphs, wherein the health monitor includes two electrodes configured to be electrically connected to the subject using a removable adhesive.

19. The health monitor of any of the preceding paragraphs, wherein the accelerometer comprises a three-axis accelerometer.

20. A method for determining fitness metrics for a subject by a health monitor configured to be supported by the subject, the method comprising:

receiving, by a processor, motion data that was generated by the health monitor in response to a first activity performed by the subject;
receiving, from a cardiac sensor in communication with the processor, cardiac data for the subject detected during performance of the first activity; and
calculating a first caloric burn rate for the first activity performed by the subject according to a first algorithm based upon information in both the motion data and cardiac data.

21. The method of paragraph 20, further comprising executing a power conservation mode of the health monitor based upon data received from the cardiac sensor.

22. The method of paragraphs 20 or 21, further comprising reducing power to at least the cardiac sensor for an interval between T-wave and P-wave portions of successive cardiac cycles.

23. The method of any of paragraphs 20-22, wherein the calculation of the first caloric burn rate includes values of heart rate and speed of the subject or values of heart rate and foot contact time of the subject.

24. The method of any of paragraphs 20-23, wherein the calculation of the first caloric burn rate includes values of heart rate and a $VO_2$ max value computed for the subject.

25. The method of any of paragraphs 20-24, further comprising calibrating a second algorithm for determining caloric burn for a subject performing an activity, wherein the calibration is based upon results obtained from the first algorithm.

26. The method of paragraph 25, wherein the second algorithm comprises determining caloric burn based upon cardiac data and not based upon motion data.

27. The method of any of paragraphs 20-26, further comprising:

calculating a second caloric burn rate for a second activity performed by the subject for a first time interval of a sequence of first time intervals; and
determining whether the second caloric burn rate falls within at least one recommended range of values proscribed by a health entity for the duration of the first time interval.

28. The method of paragraph 27, wherein the health entity is the Center for Disease Control or the World Health Organization.

29. The method of paragraphs 27 or 28, further comprising:

determining a power spectrum for the motion data; and

verifying performance of the second activity from the power spectrum.

30. The method of any of paragraphs 27-29, further comprising:

recording a first credit value for each first time interval that the second caloric burn rate falls within a first recommended range of the at least one recommended range for the duration of the first time interval; and recording a second credit value for each first time interval that the second caloric burn rate falls within a second recommended range of the at least one recommended range for the duration of the first time interval.

31. The method of paragraph 30, wherein the first recommended range of the at least one recommended range is between approximately 3.5 kilocalories per minute and approximately 7 kilocalories per minute and the second recommended range of the at least one recommended range includes values greater than approximately 7 kilocalories per minute.

32. The method of paragraphs 30 or 31, further comprising identifying a type of the second activity performed by the subject from among a plurality of activity types for which the health monitor is configured to recognize.

33. The method of any of paragraphs 20-32, further comprising:

determining, from the motion data, a period of increased activity performed by the subject; and determining a heart-rate recovery time following cessation of the activity.

34. The method of any of paragraphs 20-33, further comprising calculating heart rate variability or a LF/HF spectral power ratio from the cardiac data.

35. A computer-readable storage medium including machine-readable instructions that, when executed by at least one processor of a health monitor that includes a cardiac sensor and accelerometer, adapt the health monitor to:

receive, by the processor, motion data that was generated by the accelerometer in response to a first activity performed by a subject; receive cardiac data for the subject detected during performance of the first activity; and calculate a first caloric burn rate for the first activity performed by the subject according to a first algorithm based upon information in both the motion data and cardiac data.

36. The computer-readable storage medium of paragraph 35, further comprising machine-readable instructions that, when executed by the at least one processor, adapt the health monitor to:

calculate a second caloric burn rate for a second activity performed by the subject for a first time interval of a sequence of first time intervals; and determine whether the second caloric burn rate falls within at least one recommended range of values proscribed by a health entity for the duration of the first time interval.

37. The computer-readable storage medium of paragraph 36, further comprising machine-readable instructions that, when executed by the at least one processor, adapt the health monitor to:

record a first credit value for each first time interval that the second caloric burn rate falls within a first recommended range of the at least one recommended range for the duration of the first time interval; and record a second credit value for each first time interval that the second caloric burn rate falls within a second recommended range of the at least one recommended range for the duration of the first time interval.

**Claims**

1. A health monitor configured to be supported by a subject, the health monitor comprising:

an accelerometer configured to generate motion data in response to an activity performed by the subject over a sequence of time intervals; a cardiac sensor configured to detect at least a heart rate of the subject during performance of the activity over the sequence of time intervals; an on-board power source configured to provide power to the accelerometer and the cardiac sensor; at least one processor; and

a computer-readable medium that includes machine-readable instructions that, when executed by the at least one processor, adapts the health monitor to:

receive motion data that was generated by the accelerometer in response to the activity performed by a subject over the sequence of time intervals;
receive cardiac data for the subject detected during performance of the activity over the sequence of time intervals;
calculate a caloric burn rate for the activity performed by the subject over each time interval according to an algorithm based upon information in both the motion data and cardiac data;
record a first credit value for each time interval that the caloric burn rate falls within a first recommended range for the duration of that time interval; and
record a second credit value for each time interval that the caloric burn rate falls within a second recommended range for the duration of that time interval.

2. The health monitor of claim 1, wherein the calculation of the caloric burn rate comprises using values of heart rate and speed of the subject or values of heart rate and foot contact time of the subject.

3. The health monitor of claim 1, wherein the calculation of the caloric burn rate comprises using values of heart rate and a V02 max value computed for the subject.

4. The health monitor of any preceding claim, wherein the first recommended range is between approximately 3.5 kilocalories per minute and approximately 7 kilocalories per minute and the second recommended range is greater than approximately 7 kilocalories per minute.

5. The health monitor of any preceding claim, wherein the computer-readable medium includes machine-readable instructions that, when executed by the at least one processor, adapts the health monitor to:

determine, from data received from the accelerometer, a period of increased activity performed by the subject; and
determine a heart-rate recovery time following cessation of the activity.

6. The health monitor of any preceding claim, wherein the computer-readable medium includes machine-readable instructions that, when executed by the at least one processor, adapts the health monitor to determine at least one power conservation mode of the health monitor based upon data received from the cardiac sensor, wherein the at least one power conservation mode comprises a mode in which power is reduced to at least the cardiac sensor for an interval between T-wave and P-wave portions of successive cardiac cycles.

7. The health monitor of any preceding claim comprising an on-board memory for storing the first and second credit values.

8. A method for operating a health monitor comprising:

receiving motion data generated by an accelerometer in response to an activity performed by a subject over a sequence of time intervals;
receiving cardiac data for the subject detected during performance of the activity over the sequence of time intervals;
calculating a caloric burn rate for the activity performed by the subject over each time interval according to an algorithm based upon information in both the motion data and cardiac data;
recording a first credit value for each time interval that the caloric burn rate falls within a first recommended range for the duration of that time interval; and
recording a second credit value for each time interval that the caloric burn rate falls within a second recommended range for the duration of that time interval.

9. The method of claim 8, wherein calculating the caloric burn rate comprises using values of heart rate and speed of the subject or values of heart rate and foot contact time of the subject.

10. The method of claim 8, wherein calculating the caloric burn rate comprises using values of heart rate and a V02 max value computed for the subject.

11. The method of any one of claims 8 to 10, wherein the first recommended range is between approximately 3.5 kilocalories per minute and approximately 7 kilocalories per minute and the second recommended range is greater than approximately 7 kilocalories per minute.

12. The method of any one of claims 8 to 10 comprising:

determining, from data received from the accelerometer, a period of increased activity performed by the subject; and
determining a heart-rate recovery time following cessation of the activity.

13. The method of any one of claims 8 to 10 comprising executing a power conservation mode of the health monitor based upon data received from the cardiac sensor, wherein the power conservation mode comprises a mode in which power is reduced to at least the cardiac sensor for an interval between T-wave and P-wave portions of successive cardiac cycles.

14. The method of any one of claims 8 to 10 comprising storing the first and second credit values on an on-board memory of the health monitor.

15. A computer-readable medium that includes machine-readable instructions that, when executed by at least one processor of a health monitor, adapts the health monitor to:

receive motion data that was generated by the accelerometer in response to the activity performed by a subject over the sequence of time intervals;
receive cardiac data for the subject detected during performance of the activity over the sequence of time intervals;
calculate a caloric burn rate for the activity performed by the subject over each time interval according to an algorithm based upon information in both the motion data and cardiac data;
record a first credit value for each time interval that the caloric burn rate falls within a first recommended range for the duration of that time interval; and
record a second credit value for each time interval that the caloric burn rate falls within a second recommended range for the duration of that time interval.

100

170

*FIG. 1A*

185

182

180

105

172

174

*FIG. 1B*

battery
105

motion
sensor
152

wake-up
150

accelerometer
130

biophysical
sensor
154

MCU
110

memory
120

transceiver
140

*FIG. 1C*

*FIG. 1D*

*FIG. 1E*

104

106   186   187

## FIG. 1F

200

280   sleep mode
210   220

step
detect
270   215   wake
qualify
230

260   step
qualify
250   240

## FIG. 2A

160

160-1

160-2

160-3

160-5

160-4

160-7

160-6

160-8

160-9

160-10

160-11

*FIG. 1G*

*FIG. 2B*

*FIG. 2C*

206

receive full
ECG signal
226

determine timing of
PQRST wave
228

awake before
R wave
252-1

determine R wave
point
252-3

HM sleep
232-9

N          R wave point valid?          Y
252-5

## FIG. 2D

R          R-R Interval          R

P          T

Q   S

## FIG. 2E

*FIG. 2F*

FIG. 3

activity data: walking

FIG. 4A

activity data: biking

FIG. 4B

FIG. 5A

FIG. 5B

FIG. 5C

activity
data

600

identify activity
(known, unknown, non-human)
610

Y    activity continued
for minimum creditable
time?
630    N

determine health
credit and add to
activity buffer
660

add null value to
activity buffer
640

N    activity buffer
full?
670    Y

copy buffer and
analyze
680

store activity
summary
690

*FIG. 6*

610

```
┌─────────────────┐
│ receive activity data │
│        612        │
└─────────────────┘
         │
         ▼
┌─────────────────┐
│ process activity data │◄────┐
│        614        │      │
└─────────────────┘      │
         │               │
         ▼               │
      N ╱ activity ╲ Y   │
   ◄────╱ non-human? ╲───┘
        ╲    616    ╱
         ╲        ╱
         │
         ▼
    Y ╱ activity  ╲ N
  ◄──╱ recognizable? ╲──────┐
     ╲     618     ╱        │
      ╲          ╱          │
     │                      ▼
     │              Y ╱ similar to ╲ N
     │            ◄──╱ recognizable ╲──┐
     │               ╲  activity?  ╱   │
     │                ╲    619   ╱     │
     ▼               │                 ▼
┌──────────┐         ▼           ┌──────────┐
│ identify  │   ┌──────────┐     │  assign   │
│activity type│ │ identify  │    │unknown type│
│    620    │   │activity type│   │    622    │
└──────────┘   │    620    │     └──────────┘
     │         └──────────┘           │
     ▼              │                 ▼
┌──────────┐        ▼           ┌──────────┐
│ determine │  ┌──────────┐     │  assign   │
│intensity value│ │ determine │  │intensity value│
│    625    │  │intensity value│ │    627    │
└──────────┘   │    626    │     └──────────┘
     │         └──────────┘           │
     │              │                 │
     └──────────────┼─────────────────┘
                    ▼
                ╭────────╮
                │   630  │
                ╰────────╯
```

*FIG. 7A*

660

```
receive activity
type data
662
        │
        ▼
look up activity type
METs
664
        │
        ▼
compute caloric
burn rate
665
```

```
determine
health credit value
667
        │
        ▼
add health credit value
and activity type to activity
buffer
669
        │
        ▼
      670
```

## FIG. 7B

660-a

```
receive activity data
for running or walking
662-a
        │
        ▼
determine current VO₂
max for subject
662-b
        │
        ▼
receive activity data
664-a
        │
        ▼
determine caloric
burn rate for activity
665-a
```

```
determine
health credit value
667
        │
        ▼
add health credit value
and activity type to activity
buffer
669
        │
        ▼
      670
```

## FIG. 7C

802

| activity type 810 | intensity 812 | METs 814 | |
|---|---|---|---|
| 0110 | 00110 | 01000 | running, 4-6 mph |
| 0110 | 01000 | 01011 | running, 6-8 mph |
| 0110 | 01010 | 01101 | running, 8-10 mph |
| 0110 | 01100 | 10000 | running, 10-12 mph |
| 0110 | 01110 | 10101 | running, 12-14 mph |

⋮

| 1011 | 00000 | 00101 | aerobics, low impact |
| 1011 | 00001 | 00111 | aerobics, high impact |

⋮

| 0001 | 00000 | 00001 | resting |

⋮

*FIG. 8A*

health credit    activity type

data entry
820

805

01    0110    01    0110    10    0110         00    0001

M bits    N bits

*FIG. 8B*

| start date/time 850 | health credits 852-1 | enhanced credits 854-1 | activity 856-1 | health credits 852-2 | enhanced credits 854-2 | activity 856-2 | |
|---|---|---|---|---|---|---|---|

Q bits    R bits    S bits    T bits

on-board stored
data stream
860

*FIG. 8C*

912       914              910

| 01 | 0110 | 01 | 0110 | 10 | 0110 | 10 | 0110 | 01 | 0110 |

916

| 00 | 0011 | 00 | 0001 | 00 | 0110 | 00 | 0110 | 00 | 0011 |

918             920     922

| 00 | 0000 | 00 | 0001 | 00 | 0000 | 11 | 0000 | 01 | 1001 |

924

| 10 | 1001 | 10 | 1001 | 00 | 0001 | 10 | 1001 | 01 | 1001 |

*FIG. 9A*

930

| health credits 852-n | enhanced credits 854-n | activity 856-n |
|---|---|---|
| 001111 | 0000 | 1001 |

950

| carry time 952 | carry credits 954 |
|---|---|
| 00101 | 01000 |

*FIG. 9B*

walking: monitor on ankle

*FIG. 10A*

walking: monitor on belt

*FIG. 10B*

walking: monitor in pocket

*FIG. 10C*

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | R. M. GOW ET AL: "Activity Intensity During Free-Living Activities in Children and Adolescents With Inherited Arrhythmia Syndromes: Assessment by Combined Accelerometer and Heart Rate Monitor", CIRCULATION. ARRHYTHMIA AND ELECTROPHYSIOLOGY, vol. 6, no. 5, 1 October 2013 (2013-10-01), pages 939-945, XP055491166, United States ISSN: 1941-3149, DOI: 10.1161/CIRCEP.113.000514 * page 939, column 2 * * section "Rationale and Methods: Activity Assessment" * * section "Rationale and Methods: Accelerometer" * * section "Rationale and Methods: Individual Calibration" * * section "Rationale and Methods: Study Protocol" * * section "Rationale and Methods: Accelerometer and Heart Rate Event Definitions" * * section "Results: Event Data" * ----- | 1-15 | INV. A61B6/00 A61B5/22 G06F19/00 |
| X | US 2013/332286 A1 (MEDELIUS PEDRO J [US] ET AL) 12 December 2013 (2013-12-12) * paragraph [0022] - paragraph [0029] * * paragraph [0035] - paragraph [0039] * * paragraph [0044] * * paragraph [0092] - paragraph [0093] * * paragraph [0099] * * paragraph [0111] - paragraph [0114] * * paragraph [0141] * * paragraph [0146] * ----- -/-- | 1-15 | TECHNICAL FIELDS SEARCHED (IPC) A61B A63B G06F |

The present search report has been drawn up for all claims

| Place of search | Date of completion of the search | Examiner |
|---|---|---|
| The Hague | 9 July 2018 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 16 8090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 849 504 A1 (POLAR ELECTRO OY [FI]) 31 October 2007 (2007-10-31) <br> * paragraph [0012] * <br> * paragraph [0018] - paragraph [0022] * <br> * paragraph [0051] * <br> * paragraph [0060] - paragraph [0062] * <br> * figure 3 * <br> ----- | 1-15 | |
| A | US 2007/276271 A1 (CHAN RAYMOND [HK]) 29 November 2007 (2007-11-29) <br> * paragraph [0089] - paragraph [0091] * <br> ----- | 2,3,9,10 | |
| A | EP 2 095 763 A1 (POLAR ELECTRO OY [FI]) 2 September 2009 (2009-09-02) <br> * paragraph [0052] * <br> ----- | 5,12 | |

TECHNICAL FIELDS
SEARCHED     (IPC)

The present search report has been drawn up for all claims

| Place of search | Date of completion of the search | Examiner |
|---|---|---|
| The Hague | 9 July 2018 | Gooding Arango, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 18 16 8090

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2018

| Patent document cited in search report | | Publication date | Patent family member(s) | | Publication date |
|---|---|---|---|---|---|
| US 2013332286 | A1 | 12-12-2013 | US 2013332286 A1 | | 12-12-2013 |
| | | | WO 2012115943 A1 | | 30-08-2012 |
| EP 1849504 | A1 | 31-10-2007 | AT 414557 T | | 15-12-2008 |
| | | | EP 1849504 A1 | | 31-10-2007 |
| | | | ES 2319339 T3 | | 06-05-2009 |
| | | | FI 20065259 A | | 25-10-2007 |
| | | | US 2007249470 A1 | | 25-10-2007 |
| | | | US 2010197403 A1 | | 05-08-2010 |
| US 2007276271 | A1 | 29-11-2007 | CN 101061949 A | | 31-10-2007 |
| | | | EP 1849407 A1 | | 31-10-2007 |
| | | | ES 2445183 T3 | | 28-02-2014 |
| | | | HK 1103613 A1 | | 07-03-2014 |
| | | | US 2007276271 A1 | | 29-11-2007 |
| EP 2095763 | A1 | 02-09-2009 | EP 2095763 A1 | | 02-09-2009 |
| | | | US 2009216143 A1 | | 27-08-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 84009813 A
- US 69031312 A
- US 61566528 A
- US 4578769 A
- US 840098 A

**Non-patent literature cited in the description**

- **BARBARA E. AINSWORTH et al.** 2011 Compendium of Physical Activities: A Second Update of Codes and MET Values. American College of Sports Medicine
- **WEYAND, P. G. et al.** *J. Appl. Physiol.,* 2001, vol. 91, 451-458